# FASCICULE DE BREVET EUROPEEN

(11) **EP 3 645 726 B1**
(45) Date de publication et mention de la délivrance du brevet: **11.11.2026**
(21) Numéro de dépôt: 18750248.9
(22) Date de dépôt: 29.06.2018
(51) Int. Cl.: C12N 15/81

(54) **UTILISATION DES POLYKÉTIDE SYNTHASES DE TYPE III DE BACTÉRIES COMME PHLOROGLUCINOL SYNTHASES**
VERWENDUNG VON TYP-III-POLYKETIDSYNTHASEN AUS BAKTERIEN ALS PHLOROGLUCINSYNTHASEN
USE OF TYPE III POLYKETIDE SYNTHASES FROM BACTERIA AS PHLOROGLUCINOL SYNTHASES

(30) Priorité: 30.06.2017 FR 1756106
(43) Date de publication de la demande: 06.05.2020
(73) Titulaire: Compagnie Générale des Etablissements Michelin, 63000 Clermont-Ferrand (FR)
(72) Inventeur: LAFAQUIERE, Vincent, 63040 Clermont Ferrand Cedex 9 (FR); RAMAEN, Odile, 78660 Ablis (FR); LOUIS, Dominique, 91470 Forges Les Bains (FR)
(74) Mandataire: M.F.P. Michelin
(86) Numéro de dépôt international: PCT/FR2018/051619
(87) Numéro de publication internationale: WO 2019/002799

(56) Documents cités:
- US-A1- 2014 315 269
- DATABASE UniProt [online] 12 April 2017 (2017-04-12), "SubName: Full=Long-chain alpha-pyrone synthase {ECO:0000313|EMBL:SDQ98350.1};", XP002780022, retrieved from EBI accession no. UNIPROT:A0A1H1FBR5 Database accession no. A0A1H1FBR5
- DATABASE UniProt [online] 8 June 2016 (2016-06-08), "SubName: Full=Polyketide synthase {ECO:0000313|EMBL:KXP15269.1};", XP002780023, retrieved from EBI accession no. UNIPROT:A0A138AXW3 Database accession no. A0A138AXW3
- DATABASE Protein [online] 19 August 2016 (2016-08-19), "polyketide synthase [Tsukamurella tyrosinosolvens]", XP002780024, retrieved from NCBI accession no. RefSeq:WP_068743031.1 Database accession no. WP_068743031.1
- L. MESLET-CLADIERE ET AL: "Structure/Function Analysis of a Type III Polyketide Synthase in the Brown Alga Ectocarpus siliculosus Reveals a Biochemical Pathway in Phlorotannin Monomer Biosynthesis", THE PLANT CELL, vol. 25, no. 8, 1 August 2013 (2013-08-01), US, pages 3089 - 3103, XP055466135, ISSN: 1040-4651, DOI: 10.1105/tpc.113.111336
- IKURO ABE ET AL: "Engineered Biosynthesis of Plant Polyketides: Chain Length Control in an Octaketide-Producing Plant Type III Polyketide Synthase", JOURNAL OF THE AMERICAN CHEMICAL SOCIETY, AMERICAN CHEMICAL SOCIETY, vol. 127, no. 36, 14 September 2005 (2005-09-14), pages 12709 - 12716, XP002752480, ISSN: 0002-7863, [retrieved on 20050817], DOI: 10.1021/JA053945V
- ACHKAR JIHANE ET AL: "Biosynthesis of phloroglucinol", JOURNAL OF THE AMERICAN CHEMICAL SOCIETY, AMERICAN CHEMICAL SOCIETY, vol. 127, no. 15, 26 March 2005 (2005-03-26), pages 5332 - 5333, XP002397679, ISSN: 0002-7863, DOI: 10.1021/JA042340G
- WENJUAN ZHA ET AL: "Characterization of the Substrate Specificity of PhlD, a Type III Polyketide Synthase from Pseudomonas fluorescens", JOURNAL OF BIOLOGICAL CHEMISTRY, vol. 281, no. 42, 24 August 2006 (2006-08-24), US, pages 32036 - 32047, XP055388856, ISSN: 0021-9258, DOI: 10.1074/jbc.M606500200
- HARIYANTI BAHARUM ET AL: "Molecular Cloning, Modeling, and Site-Directed Mutagenesis of Type III Polyketide Synthase from(Phaeophyta)", MARINE BIOTECHNOLOGY, SPRINGER-VERLAG, NE, vol. 13, no. 5, 23 December 2010 (2010-12-23), pages 845 - 856, XP019939493, ISSN: 1436-2236, DOI: 10.1007/S10126-010-9344-5

## Description

### DOMAINE DE L'INVENTION

La présente invention se situe dans les domaines de la biochimie microbienne et plus particulièrement dans le domaine de la synthèse par des enzymes microbiennes du phloroglucinol. Elle concerne l'utilisation de polykétide synthases de type III de bactéries actinomycètes particulières, comme phloroglucinol synthases.

### ART ANTERIEUR

Le phloroglucinol est un composé organique aromatique utilisé notamment dans la fabrication de produits pharmaceutiques et d'explosifs.

La synthèse de phloroglucinol est catalysée par des polykétide synthases de type III appelées phloroglucinol synthases. Les phloroglucinol synthases réalisent la condensation de trois molécules de malonyl-CoA pour former une molécule de phloroglucinol selon le schéma réactionnel suivant (Réaction I):

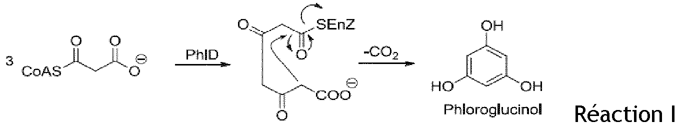

De nombreux oligomères peuvent ensuite être synthétisés à partir du phloroglucinol, tels que les phlorotannins. Les phlorotannins incluent notamment les fucols, les phloretols et les fucophloretols, qui sont des produits dérivés du phloroglucinol composant la paroi des algues brunes. En outre, diverses activités protectrices des algues brunes ont également été attribuées aux phlorotannins.

Les bases de données Uniprot ou NCBI comprennent des séquences pour lesquelles l'annotation mentionne plusieurs activités putatives, donc non vérifiées, dont une activité putative de polykétide synthases de type III (PKS III) (telles que les séquences identifiées par les numéros Uniprot A0A1H1FBR5 (décrite dans XP-002780022) ou A0A138AXW3 (décrite dans XP-002780023), ou le numéro NCBI WP_068743031 (décrite dans XP-002780024)).

Abe et al., 2005 (Engineered Biosynthesis of Plant polyketides : Chain Length Control in an Octaketide-Producing Plant Type III Polyketide Synthase, J. Am. Chem. Soc., 127, 12709-12716) décrit un gène codant une PKS III chez la plante *Aloe arborescens* présentant une activité d'octakétide synthase. L'activité de phloroglucinol synthase n'est pas mentionnée.

Baharum et al., 2011 (Molecular Cloning, Modeling, and Site-Directed Mutagenesis of Type III Polyketide Synthase from Sargassum Binderi ; Mar Biotechnol, 13 :845-856) décrit un gène codant une PKS III chez l'algue brune *Sargassum binderi*. Les auteurs montrent que cette PKS III ne présente aucune activité de phloroglucinol synthase.

A ce jour, la synthèse du phloroglucinol a été décrite uniquement chez les bactéries Gram-*Pseudomonas fluorescens* (Achkar et al., 2005 ; Zha et al., 2006) et chez l'algue brune *Ectocarpus siliculosus* (Meslet-Cladière et al., 2013). L'enzyme phloroglucinol synthase impliquée dans la synthèse du phloroglucinol a été identifiée chez ces deux espèces. Ce sont les deux seules phloroglucinol synthases identifiées et caractérisées à ce jour.

Chez *Pseudomonas fluorescens,* la phloroglucinol synthase est codée par le gène **PHLD** (Achkar et al., 2005 ; Zha et al., 2006).

Chez *Ectocarpus siliculosus,* la phloroglucinol synthase est codée par le gène PKS1 (Meslet-Cladière et al., 2013 ; US2014/315269).

L'activité de phloroglucinol synthase de **PHLD** a pu être démontrée chez *Escherichia coli* exprimant un gène **PHLD** hétérologue (Achkar et al., 2005). Cette activité a été confirmée in *vitro*, par des tests enzymatiques à petite échelle réalisés avec une **PHLD** recombinante exprimée et purifiée à partir de cultures d'*Escherichia coli* (Zha et al., 2006).

L'activité de phloroglucinol synthase de **PKS1** a été démontrée *in vitro,* à partir de PKS1 recombinante exprimée et purifiée chez *Escherichia* coli et à partir d'extraits cellulaires d'*E*. *siliculosus* (Meslet-Cladière et al., 2013, WO 2013/045510).

Toutefois, les enzymes PHLD et PKS1 présentent des activités enzymatiques faibles. En outre, la possibilité de synthétiser le phloroglucinol *in vitro* à grande échelle en utilisant ces enzymes n'a jamais été prouvée. Enfin, les phloroglucinol synthases utilisées dans ces travaux ont été produites par des bactéries *E. coli* ou *P. fluorescens*. L'activité de ces enzymes lorsqu'elles sont produites par des eucaryotes, tels que des levures ou des cellules d'insectes ou de mammifères, n'est ainsi pas connue. Or, les systèmes eucaryotes peuvent être avantageux, notamment pour des productions à grande échelle. Ils permettent en effet l'obtention d'enzymes pouvant être modifiées au niveau post-traductionnel.

Ainsi, il existe toujours le besoin d'identifier de nouvelles phloroglucinol synthases présentant une activité enzymatique *in vitro* ou *in vivo* élevée de synthèse de phloroglucinol, adaptées pour une production à l'échelle industrielle et pouvant être produites par des systèmes eucaryotes.

La caractérisation fonctionnelle exacte d'une polykétide synthase est cependant compliquée par le fait que cette classe d'enzymes rassemble des protéines présentant de grandes similarités de séquences alors qu'elles peuvent catalyser des réactions sensiblement dissemblables et reconnaître des substrats tout à fait différents.

Malgré ces difficultés, les présents Inventeurs ont pu identifier des polypeptides présents chez les bactéries ayant une activité phloroglucinol synthase. Ainsi, de nouvelles phloroglucinol synthases ont notamment été identifiées chez des bactéries Gram+. Elles constituent le premier exemple de phloroglucinol synthases chez ce type de bactéries. Les Inventeurs démontrent ici que ces nouvelles phloroglucinol synthases présentent une activité de synthèse de phloroglucinol élevée. Elles sont donc adaptées à la production à l'échelle industrielle. De plus, elles sont fonctionnelles lorsqu'elles sont produites en système eucaryote.

### RESUME DE L'INVENTION

Dans le cadre de la présente invention, les Inventeurs ont mis en évidence, de manière tout à fait surprenante, que des bactéries, outre *Pseudomonas fluorescens,* contiennent dans leur génome un gène codant une polykétide synthase de type III ayant une activité de phloroglucinol synthase fonctionnelle.

La présente invention est telle que définie dans les revendications.

La présente invention se rapporte donc à l'utilisation d'au moins un polypeptide choisi parmi les polyketide synthases de type III de bactéries actinomycètes, comme phloroglucinol synthase ; ledit polypeptide comprenant au moins une séquence d'acides aminés ayant au moins 60% d'identité avec une séquence choisie parmi SEQ ID NO: 4, SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 9 et SEQ ID NO: 10.

Il est donc ici décrit des polypeptides choisis parmi les polykétide synthases de type III de bactéries qui ne figurent pas dans le texte des revendications, notamment parmi les polykétide synthases de type III de bactéries gram+, notamment les polykétide synthases de type III de bactéries actinomycètes, utiles comme phloroglucinol synthases.

Sont également décrites des molécules d'acides nucléiques isolées comprenant au moins une séquence d'acides nucléiques codant pour un polypeptide choisi parmi les polyketide synthases de type III de bactéries actinomycètes telles que décrites ici, et comprenant en outre un promoteur et/ou un terminateur contrôlant l'expression de ladite au moins une séquence d'acides nucléiques.

Sont également décrits, mais non définis dans les revendications, des vecteurs comprenant au moins une molécule d'acides nucléiques isolée telle que définie ci-dessus, codant pour une telle phloroglucinol synthase.

L'invention concerne également des cellules hôtes comprenant au moins une molécule d'acides nucléiques isolée ou au moins un vecteur comprenant au moins une molécule d'acides nucléiques isolée, la molécule d'acides nucléiques isolée comprenant au moins une séquence d'acides nucléiques codant un polypeptide selon l'invention et comprenant en outre un promoteur contrôlant l'expression de ladite au moins une séquences d'acides nucléiques, et la cellule hôte étant un microorganisme sélectionné parmi les levures.

Il est également ici décrit des méthodes pour produire une phloroglucinol synthase fonctionnelle, qui ne figurent pas dans le texte des revendications.

L'invention concerne également des méthodes pour produire du phloroglucinol, telles que définies dans les revendications.

### DESCRIPTION DES FIGURES

La **Figure 1** présente l'alignement des séquences protéiques des enzymes candidates identifiées. L'alignement des dix enzymes sélectionnées avec l'enzyme PKS1.Es (PHLD.Es) a été réalisé en employant le logiciel Clustal W.
La **Figure 2** présente un exemple de séparation du phloroglucinol / résorcinol sur colonne propyl-pentafluorophenyl (PFP).
La **Figure 3** montre un exemple de la structure d'une unité génique construite pour un candidat donné (PhID.ii), permettant d'exprimer les gènes PHLD chez la levure *Saccharomyces cerevisiae.*

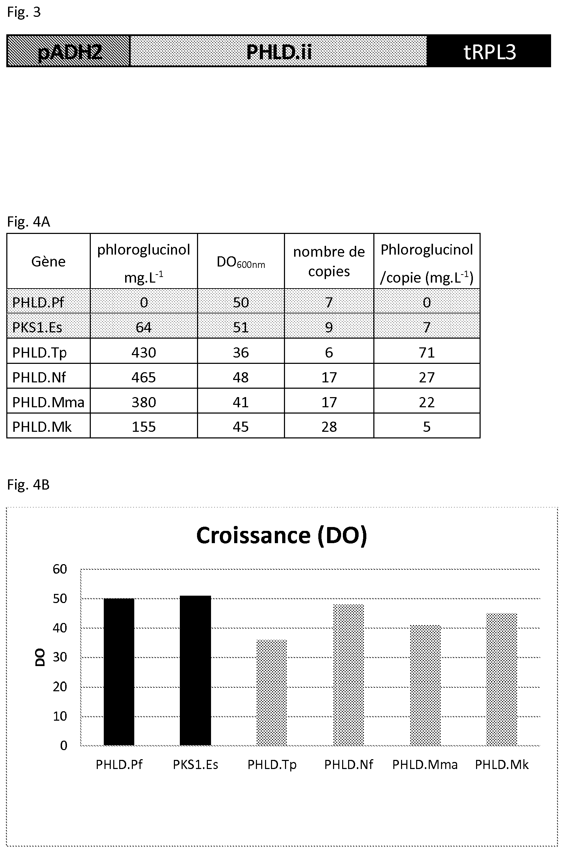

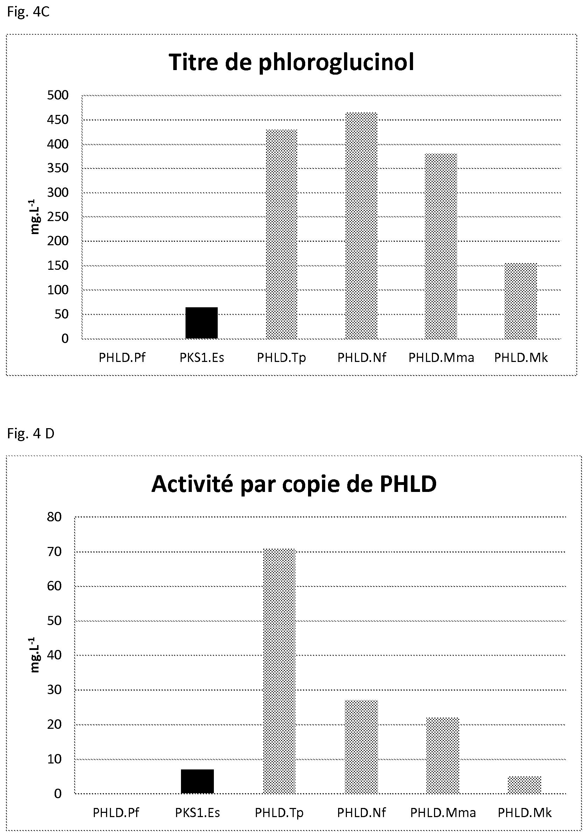

La **Figure 4** montre les niveaux de production de phloroglucinol dans les souches de levure exprimant les différents gènes candidats PHLD.ii et les gènes contrôles PHLD.Pf et PKS1.Es (plusieurs copies) sous le contrôle du promoteur ADH2, après 48 heures de culture en plaque 24 puits en présence de 20 g.L⁻¹ d'éthanol comme source de carbone à 30°C. (A) Récapitulatif des différentes données mesurées. (B) Densités optiques (DO) des différentes cultures mesurées à 600 nm (DO₆₀₀), indiquant le niveau de croissance de chaque souche. (C) Niveau de production de phloroglucinol (en mg.L⁻¹) mesuré dans le milieu de culture. (D) Niveau de production de phloroglucinol (en mg.L⁻¹) normalisé par rapport au nombre de copies de gènes intégrées dans le génome.
La **Figure 5** montre la structure des constructions géniques intégrées dans le génome de la levure au locus JLP1. Le gène codant pour chaque PHLD/PKS1 est sous contrôle du promoteur pADH2 ou du promoteur pCCW12.
La **Figure 6** montre les niveaux de production de phloroglucinol dans les souches de levure exprimant les différents gènes PHLD.ii (1 seule copie) sous le contrôle du promoteur ADH2 après 48 heures de culture en plaque 24 puits en présence de 20 g.L⁻¹ d'éthanol comme source de carbone à 30°C. (A) Récapitulatif des différentes données mesurées. (B) Densités optiques (DO) des différentes cultures mesurées à 600 nm (DO₆₀₀), indiquant le niveau de croissance de chaque souche. (C) Niveau de production de phloroglucinol (en mg.L⁻¹) mesuré dans le milieu de culture.
La **Figure 7** montre les niveaux de production de phloroglucinol dans les souches de levure exprimant les différents gènes PHLD (1 seule copie) sous le contrôle du promoteur CCW12 après 48 heures de culture en plaque 24 puits en présence de 20 g.L⁻¹ de glucose comme source de carbone à 30°C. (A) Récapitulatif des différentes données mesurées. (B) Densités optiques (DO) des différentes cultures mesurées à 600 nm (DO₆₀₀), indiquant le niveau de croissance de chaque souche. (C) Niveau de production de phloroglucinol (en mg.L⁻¹) mesuré dans le milieu de culture.

### DESCRIPTION DETAILLEE DE L'INVENTION

### Définitions

Par « **polykétide synthase de type III** », on entend une enzyme multifonctionnelle ou un complexe enzymatique produisant des polykétides et qui n'utilise pas de domaine de protéine porteuse d'acyle (ou ACP, pour « acyl carrier protein »).

Par « **polykétide** », on entend une grande famille de métabolites secondaires chez les bactéries, les mycètes, les plantes et certaines lignées d'animaux qui proviennent de la condensation itérative de sous-unités acétyle ou malonyle par des enzymes polykétide-synthases. Les polykétides servent également de matières premières pour la fabrication d'un éventail large de produits naturels et semi-synthétiques.

Par « **phloroglucinol** », on entend un composé organique aromatique **benzène-1,3,5-triol** présentant la formule chimique suivante (Formule I) :

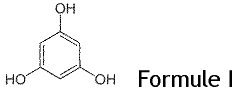

Par « **phloroglucinol synthase** », on entend une enzyme multifonctionnelle ou un complexe enzymatique appartenant à la famille des **polykétide synthases de type III** et catalysant la synthèse du phloroglucinol. Une phloroglucinol synthase catalyse la condensation de trois molécules de malonyl-CoA pour former une molécule de phloroglucinol.

Par « **activité enzymatique** » ou « **activité catalytique** » ou encore « **activité** » d'une enzyme, on entend l'efficacité d'une enzyme à convertir un substrat en produit dans un environnement donné. L'efficacité de l'enzyme prend en compte ici la vitesse de conversion du substrat en produit par l'enzyme et le taux de conversion du substrat en produit par l'enzyme. Par « **taux de conversion du substrat en produit par l'enzyme** » on entend ici le ratio entre la quantité de produit final obtenu par rapport à la quantité initiale de substrat pour une quantité définie d'enzyme. Par exemple, une activité enzymatique au sens de l'invention peut être exprimée en quantité de phloroglucinol produit dans un volume donné (en g/L).

Par « **bactérie** », on entend un organisme microscopique et procaryote présent dans tous les milieux.

Par « **bactérie Gram+** » ou « **bactérie Gram-positive** », on entend une bactérie positive à la coloration de Gram (c'est-à-dire qui retient le violet de gentiane, appelé aussi le cristal violet, et reste colorée en mauve-violet ou bleu-pourpre foncé).

Par « **bactérie actinomycète** » ou « **bactérie actinomycète Gram+** » ou « **bactérie actinomycète Gram-positive** », on entend une bactérie appartenant à l'ordre des *Actinomycetales* au sein de la classe des actinobactéries, positive à la coloration de Gram.

Les actinomycètes sont des bactéries dont la croissance donne lieu à des colonies constituées d'hyphes, c'est-à-dire de filaments qui irradient, par croissance centrifuge, tout autour du germe qui leur a donné naissance.

Par « ***Tsukamurella sp*.** », on entend une bactérie actinomycète asporulée, en forme de bâtonnet et aérobie obligatoire, du genre *Tsukamurella.* Le genre *Tsukamurella* comprend notamment les espèces *Tsukamurella paurometabola* (appelée aussi Tp ci-après), *Tsukamurella tyrosinosolvens* (appelée aussi Tt ci-après), *Tsukamurella pseudospumae* (appelée aussi Tps ci-après), *Tsukamurella pulmonis* (appelée aussi Tpu ci-après) et *Tsukamurella sp.* 1534 (appelée aussi Tsp ci-après).

Par **«*Nocardia sp*.** », on entend une bactérie actinomycète filamenteuse, du genre *Nocardia.* Le genre *Nocardia* comprend notamment l'espèce *Nocardia farcinica* (appelée aussi Nf ci-après).

Par « ***Mycobacterium sp*.** », on entend une bactérie actinomycète asporulée et aérobie, en forme de bacilles, du genre *Mycobacterium.* Le genre *Mycobacterium* comprend notamment les espèces *Mycobacterium marinum*, *Mycobacterium kansasii* et *Mycobacterium tuberculosis* (appelées aussi, respectivement, Mma, Mk et Mt ci-après).

Par « ***Gordonia sp*.** », on entend une bactérie actinomycète, du genre *Gordonia.* Le genre *Gordonia* comprend notamment l'espèce *Gordonia hydrophobica* (appelée aussi Gh ci-après).

*Par* « ***Pseudomonas sp.*** », on entend une bactérie Gram négative (Gram-) ne formant pas de spores (ou asporulées), en forme de bacille et aérobie obligatoire, du genre *Pseudomonas*. Le genre *Pseudomonas* comprend notamment l'espèce *Pseudomonas fluorescens* (appelée aussi Pf ci-après).

Par « ***Ectocarpus sp.*** » on entend une algue du genre *Ectocarpus*, de la classe des algues brunes, appartenant à la famille des *Ectocarpaceae*. Le genre *Ectocarpus* comprend notamment l'espèce *Ectocarpus siliculosus.*

Par « **PHLD.Pf** », on entend indifféremment le gène codant pour la phloroglucinol synthase PHLD de *P. fluorescens*, ou le polypeptide codé par ce gène.

Par « **PKS1.Es** » ou « **PHLD.Es** », on entend indifféremment le gène codant pour la phloroglucinol synthase PKS1 d'*E. siliculosus*, ou le polypeptide codé par ce gène.

« **PhID** » ou « **PHLD** » désigne ici un gène candidat codant pour une enzyme phloroglucinol synthase candidate, ou le polypeptide codé par ce gène. Selon la nomenclature choisie par les Inventeurs, « **PhID.ii** » ou « **PHLD.ii** » désigne ici le gène candidat ou le polypeptide candidat issu d'un organisme donné. Les lettres « ii » représentent le genre et l'espèce auxquels ledit organisme appartient.

Par **«molécule d'acides nucléiques** », on entend un polymère de n'importe quelle longueur d'acide désoxyribonucléique (ADN), ou polydésoxyribonucléotides, incluant notamment les ADN complémentaires ou ADNc, les ADN génomiques, les plasmides, les vecteurs, les génomes viraux, l'ADN isolé, les sondes, les amorces et tout mélange de ceux-ci ; ou un polymère de n'importe quelle longueur d'acide ribonucléique (ARN), ou polyribonucléotides, incluant notamment les ARN messagers ou ARNm, ARN antisens ; ou des polyribo-polydésoxyribonucléotides mélangés. Ils englobent des polynucléotides simples ou à double brins, linéaires ou circulaires, naturels ou synthétiques. En outre, un polynucléotide peut comprendre des nucléotides non naturels et peut être interrompu par des composants non nucléotidiques.

Dans le cadre de la présente invention, les termes "acide nucléique", "molécule d'acides nucléiques", "polynucléotide" et "séquence nucléotidique" sont utilisés de manière interchangeable.

Par « **molécule isolée** », on entend une molécule, notamment une protéine, un polypeptide, un peptide, une molécule d'acides nucléiques, un vecteur plasmidique, un vecteur viral ou une cellule hôte, qui est extrait de son environnement naturel (c'est-à-dire séparé d'au moins un autre composant auquel il est naturellement associé).

Par « **polypeptide** », **"protéine"** et **"peptide",** on entend des polymères de résidus d'acides aminés qui comprennent au moins neuf acides aminés liés par des liaisons peptidiques. Le polymère peut être linéaire, ramifié ou cyclique. Le polymère peut comprendre des acides aminés naturels et/ou analogues d'acides aminés et il peut être interrompu par des résidus non-acides aminés. Comme indication générale et sans y être cependant lié dans la présente demande, si le polymère d'acides aminés contient plus de 50 résidus d'acides aminés, il est de préférence appelé un polypeptide ou une protéine, alors que si le polymère est constitué de 50 acides aminés ou moins, il est de préférence appelé "peptide".

Par « **vecteur** », on entend un véhicule, de préférence une molécule d'acide nucléique ou une particule virale, qui contient les éléments nécessaires pour permettre l'administration, la propagation et/ou l'expression d'une ou plusieurs molécule(s) d'acides nucléiques dans une cellule hôte ou un organisme.

D'un point de vue fonctionnel, ce terme englobe des vecteurs pour la maintenance (vecteurs de clonage), des vecteurs pour l'expression dans diverses cellules ou organismes hôtes (vecteurs d'expression), des vecteurs extrachromosomiques (par exemple des plasmides multicopie) ou des vecteurs d'intégration (par exemple conçus pour s'intégrer dans le génome d'une cellule hôte et produire des copies supplémentaires de la molécule d'acides nucléiques qu'il contient lorsque la cellule hôte se réplique). Ce terme englobe également des vecteurs navettes (par exemple, fonctionnant à la fois dans des hôtes procaryotes et/ou eucaryotes) et des vecteurs de transfert (par exemple pour le transfert de molécule(s) d'acides nucléiques dans le génome d'une cellule hôte).

D'un point de vue structurel, les vecteurs selon l'invention peuvent être des sources génétiques naturelles, synthétiques ou artificielles, ou une combinaison d'éléments génétiques naturels et artificiels.

Ainsi, dans le contexte de l'invention, le terme "vecteur" doit être compris de manière large en incluant des vecteurs plasmidiques (ou plasmides) et viraux.

Un **"plasmide"** tel qu'utilisé ici désigne une construction d'ADN réplicable. Habituellement, les vecteurs plasmidiques contiennent des gènes marqueurs de sélection qui permettent aux cellules hôtes portant le plasmide d'être identifiées et/ou sélectionnées de manière positive ou négative en présence du composé correspondant au marqueur de sélection. Une variété de gènes marqueurs de sélection positifs et négatifs sont connus dans la technique. À titre d'illustration, un gène de résistance aux antibiotiques peut être utilisé comme un gène marqueur de sélection positif permettant de sélectionner une cellule hôte en présence de l'antibiotique correspondant.

Le terme **"vecteur viral"** tel qu'utilisé ici se réfère à un vecteur d'acide nucléique qui comprend au moins un élément d'un génome du virus et peut être conditionné dans une particule virale ou une particule virale. Les vecteurs viraux peuvent être compétents pour la réplication ou sélectifs (par exemple, conçus pour se répliquer mieux ou sélectivement dans des cellules hôtes spécifiques), ou peuvent être génétiquement désactivés de manière à être défectueux ou déficients pour la réplication.

Par « **cellule hôte** », on entend une cellule contenant une molécule d'acides nucléiques selon l'invention. Avantageusement, la cellule hôte est capable d'exprimer un polypeptide à activité de phloroglucinol synthase et/ou de produire le vecteur de l'invention. Avantageusement encore, la cellule hôte est capable de synthétiser du phloroglucinol.

La cellule hôte peut être constituée d'un type unique de cellules ou d'un groupe de différents types de cellules. La cellule hôte peut également être une cellule hybride, c'est-à-dire résultant de la fusion d'au moins deux cellules de types différent.

La cellule hôte peut appartenir à des lignées cellulaires cultivées, à des cellules primaires, à des cellules souches ou à des cellules prolifératives. Dans le cadre de l'invention, la cellule hôte est un microorganisme sélectionné parmi les levures.

Le terme « cellule hôte » comprend plus largement des cellules qui contiennent ou ont contenu la molécule d'acides nucléiques selon l'invention, ainsi que la descendance de telles cellules. La cellule hôte peut par exemple être isolée ou organisée en tissu, en organe ou encore être au sein d'un organisme complet. Dans le cas où la cellule hôte est au sein d'un organisme complet, ledit organisme n'est pas humain.

Il est ainsi clair qu'une « cellule hôte » selon la présente invention est une cellule hôte recombinante, i.e., une cellule hébergeant un matériel génétique exogène. Ainsi, une cellule hôte n'est pas une cellule qui existe à l'état naturel mais est un outil de biologie moléculaire obtenu par les techniques de manipulations génétiques.

Par « **identité** », on entend une correspondance exacte de séquence entre deux polypeptides ou deux molécules d'acides aminés. Le « **pourcentage d'identité** » entre deux séquences est fonction du nombre de résidus identiques communs aux deux séquences, et prend en compte le nombre d'intervalles qui doivent être introduits pour un alignement optimal et la longueur de chaque intervalle. Divers programmes informatiques et algorithmes mathématiques sont disponibles dans l'état de l'art pour déterminer le pourcentage d'identité entre les séquences d'acides aminés, comme par exemple le programme Blast disponible sur la base NCBI ou ALIGN (Atlas of Protein Sequence and Structure, Dayhoff (ed.), 1981, Suppl. 3 482-489). Des programmes pour déterminer l'homologie entre les séquences nucléotidiques sont également disponibles dans une base de données spécialisée (par exemple Genbank, le Wisconsin Sequence Analysis Package, les programmes BESTFIT, FASTA et GAP). À titre illustratif, "au moins 80% d'identité de séquence", tel qu'utilisé ici, représente 80%, 81%, 82%, 83%, 84%, 85%, 86%, 87%, 88%, 89%, 90%, 91 %, 92%, 93%, 94%, 95%, 96%, 97%, 98%, 99% ou 100%.

Dans la description détaillée qui suit, les modes de réalisation pourront être pris seuls ou combinés de manière appropriée par l'homme du métier.

### Polypeptides isolés et leur utilisation comme phloroglucinol synthases

On s'intéresse ici aux polypeptides isolés choisis parmi les polykétide synthases de type III de bactéries, notamment parmi les polykétide synthases de type III de bactéries gram+, notamment parmi les polykétide synthases de type III de bactéries actinomycètes.

En effet, les Inventeurs ont identifié, de manière tout à fait surprenante, des gènes codant de nouvelles polykétide synthases de type III dans le génome de bactéries qui n'était pas connu pour coder ce type d'enzyme. Les Inventeurs ont notamment mis en évidence, de manière tout à fait surprenante, que ces nouvelles polykétide synthases de type III ont une activité de phloroglucinol synthase.

Il est en particulier ici décrit l'utilisation de polypeptides isolés choisis parmi les polykétide synthases de type III de bactéries comme phloroglucinol synthases, de préférence à l'exclusion de la polyketide synthase de type III de *Pseudomonas fluorescens* PHLD.Pf, qui ne figure pas dans le texte des revendications. Il est également ici décrit l'utilisation de polypeptides isolés choisis parmi les polykétide synthases de type III de bactéries comme phloroglucinol synthases, à l'exclusion de la polyketide synthase de type III de *Pseudomonas fluorescens* PHLD.Pf, qui ne figure pas dans le texte des revendications.

La présente invention concerne notamment l'utilisation de polypeptides choisis parmi les polykétide synthases de type III de bactéries actinomycètes, comme phloroglucinol synthases ; ledit polypeptide comprenant au moins une séquence d'acides aminés ayant au moins 60% d'identité avec une séquence choisie parmi SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 et SEQ ID NO: 10.

Avantageusement, ledit polypeptide isolé est choisi parmi les polykétide synthases de type III des bactéries actinomycètes choisies dans le groupe constitué par *Tsukamurella sp*., *Nocardia sp*., *Mycobacterium sp*., et *Gordonia sp*., en particulier *Tsukamurella paurometabola*, *Tsukamurella tyrosinosolvens, Tsukamurella pseudospumae, Tsukamurella pulmonis, Tsukamurella sp. 1534, Nocardia farcinica, Mycobacterium marinum, Mycobacterium kansasii, Mycobacterium tuberculosis* et *Gordonia hydrophobica*.

Ledit polypeptide comprend au moins une séquence d'acides aminés ayant au moins 60% d'identité, de préférence au moins 65% d'identité, de manière encore préférée au moins 70% d'identité, de manière encore plus préférée au moins 75% d'identité, de manière toujours plus préférée au moins 80% d'identité, de manière plus préférentielle encore au moins 85% d'identité, de manière encore plus préférentielle au moins 90% d'identité, de manière davantage préférée au moins 95% d'identité, de manière encore préférée au moins 96% d'identité, de manière encore plus préférée au moins 97% d'identité, de manière toujours plus préférée au moins 98% d'identité, et de manière plus préférentielle encore au moins 99% d'identité, avec une séquence choisie parmi SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 et SEQ ID NO: 10.

Selon un mode de réalisation particulièrement avantageux, ledit polypeptide comprend au moins une séquence d'acides aminés choisie parmi SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 et SEQ ID NO: 10.

Dans un mode de réalisation préféré, le polypeptide isolé à activité de phloroglucinol synthase possède une séquence d'acides aminés choisie parmi SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 et SEQ ID NO: 10.

Avantageusement, le polypeptide isolé à activité de phloroglucinol synthase est choisi parmi le polypeptide isolé à activité de phloroglucinol synthase PHLD.Tp de *Tsukamurella paurometabola,* le polypeptide isolé à activité de phloroglucinol synthase PHLD.Tt de *Tsukamurella tyrosinosolvens*, le polypeptide isolé à activité de phloroglucinol synthase PHLD.Tps de *Tsukamurella pseudospumae*, le polypeptide isolé à activité de phloroglucinol synthase PHLD.Tpu de *Tsukamurella pulmonis*, le polypeptide isolé à activité de phloroglucinol synthase PHLD.Tp de *Tsukamurella sp. 1534*, le polypeptide isolé à activité de phloroglucinol synthase PHLD.Nf de *Nocardia farcinica*, le polypeptide isolé à activité de phloroglucinol synthase PHLD.Mma de *Mycobacterium marinum*, le polypeptide isolé à activité de phloroglucinol synthase PHLD.Mk de *Mycobacterium kansasii*, le polypeptide isolé à activité de phloroglucinol synthase PHLD.Mt de *Mycobacterium tuberculosis* et le polypeptide isolé à activité de phloroglucinol synthase PHLD.Gh de *Gordonia hydrophobica*.

### Molécules d'acides nucléiques isolées

Sont ici décrites, mais non définies dans les revendications, des molécules d'acides nucléiques isolées comprenant au moins une séquence d'acides nucléiques codant au moins un polypeptide choisi parmi les polykétide synthases de type III de bactéries actinomycètes, et comprenant en outre un promoteur contrôlant l'expression de ladite au moins une séquence d'acides nucléiques ledit polypeptide étant tel que défini ci-dessus. Ledit polypeptide est donc un polypeptide choisi parmi les polyketide synthases de type III de bactéries actinomycètes, ledit polypeptide comprenant au moins une séquence d'acides aminés ayant au moins 60% d'identité avec une séquence choisie parmi SEQ ID NO: 4, SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 9 et SEQ ID NO: 10.

De manière avantageuse, le promoteur est un promoteur exogène, en particulier un promoteur de levure, de préférence un promoteur choisi parmi ADH2 (pADH2) et CCW12 (pCCW12), de préférence encore un promoteur choisi parmi ADH2 (pADH2) de *Saccharomyces cerevisiae* et CCW12 de *S*. *cerevisiae,* de préférence encore un promoteur choisi parmi ADH2 de SEQ ID NO: 13 et CCW12 de SEQ ID NO: 14.

Est également décrite ici mais non définie dans les revendications, une molécule d'acides nucléiques isolée comprenant au moins une séquence d'acides nucléiques codant un polypeptide choisi parmi les polykétide synthases de type III de bactéries actinomycètes tel que défini ci-dessus, et comprenant en outre un terminateur contrôlant l'expression de ladite au moins une séquence d'acides nucléiques; ledit polypeptide comprenant au moins une séquence d'acides aminés ayant au moins 60% d'identité avec une séquence choisie parmi SEQ ID NO: 4, SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 9 et SEQ ID NO: 10.

De manière avantageuse, le terminateur est un terminateur exogène, en particulier un terminateur de levure, de préférence le terminateur RPL3 (tRPL3), de préférence encore le terminateur RPL3 de *S*. *cerevisiae,* de préférence encore le terminateur RPL3 de SEQ ID NO: 15.

De préférence, la molécule d'acides nucléiques isolée comprend à la fois un promoteur et un terminateur qui sont tels que définis ci-dessus. Ainsi, est décrite ici, mais non définie dans les revendications, une molécule d'acides nucléiques isolée comprenant au moins une séquence d'acides nucléiques codant un polypeptide choisi parmi les polykétide synthases de type III tel que défini ci-dessus et comprenant en outre un promoteur et un terminateur contrôlant l'expression de ladite au moins une séquence d'acides nucléiques.

De préférence, la molécule d'acides nucléiques comprend en outre une séquence d'export. Avantageusement, cette séquence d'export permet la sécrétion ou l'excrétion du ou des polypeptides codés par ladite molécule d'acides nucléiques, dans le milieu cellulaire.

De préférence, la molécule d'acides nucléiques est isolée à partir de souches homologues en culture, de préférence choisies parmi *Tsukamurella sp*., *Nocardia sp*., et *Mycobacterium sp*., en particulier en particulier *Tsukamurella paurometabola*, *Tsukamurella tyrosinosolvens*, *Tsukamurella pseudospumae, Tsukamurella pulmonis, Tsukamurella sp. 1534, Nocardia farcinica*, *Mycobacterium marinum*, *Mycobacterium kansasii*, *Mycobacterium tuberculosis* et *Gordonia hydrophobica.*

De préférence, la molécule d'acides nucléiques est isolée à partir d'un vecteur ou d'une cellule hôte hétérologue comprenant ladite molécule, ledit vecteur ou ladite cellule hôte étant tel(le) que défini(e) plus haut et que décrit(e) ci-après dans les sections « Cellules hôtes » ou « Vecteurs ».

De préférence, la molécule d'acides nucléiques isolée est synthétisée *in vitro* par des techniques de synthèse nucléique que l'homme du métier sait parfaitement définir.

De préférence, la molécule d'acides nucléiques isolée est recombinante.

### Vecteurs

Sont ici décrits, mais non définis dans les revendications, des vecteurs comprenant au moins une molécule d'acides nucléiques telle que définie ci-dessus.

Avantageusement, le vecteur est un plasmide.

Les vecteurs qui sont appropriés comprennent, sans limitation, des vecteurs bactériophages, plasmides ou cosmides pour l'expression dans des cellules hôtes procaryotes telles que des bactéries (par exemple *E. coli*, ou des bactéries du genre *Pseudomonas*); des vecteurs pour l'expression chez la levure (par exemple *Saccharomyces cerevisiae*, *Schyzosaccharomyces pombe*, *Pichia pastoris*); des vecteurs de baculovirus pour l'expression dans des systèmes de cellules d'insectes (par exemple, des cellules de Sf 9); des vecteurs viraux et plasmidiques pour l'expression dans des systèmes de cellules végétales (par exemple le plasmide Ti, le virus de la mosaïque du chou-fleur, CaMV, le virus de la mosaïque du tabac TMV); ainsi que des vecteurs viraux et plasmidiques pour l'expression dans des cellules ou des organismes eucaryotes supérieurs.

Ces vecteurs sont généralement disponibles dans le commerce (par exemple, auprès des fournisseurs tels Invitrogen, Stratagene, Amersham Biosciences, Promega, etc.), disponibles auprès d'institutions de dépôt telles que American Type Culture Collection (ATCC, Rockville, Md.), ou ont fait l'objet de nombreuses publications décrivant leur séquence, leurs structures et leurs méthodes de production, de sorte que l'homme du métier peut les appliquer sans difficultés.

Des exemples représentatifs de vecteurs plasmidiques appropriés comprennent, sans limitation, pREP4, pCEP4 (Invitrogen), pCI (Promega), pVAX (Invitrogen) et pgWiz (Gene Therapy System Inc).

### Cellules hôtes

Dans un autre aspect, la présente invention concerne des cellules hôtes comprenant au moins une molécule d'acides nucléiques isolée ou au moins un vecteur comprenant au moins une molécule d'acide nucléiques isolée, la molécule d'acides nucléiques isolée comprenant au moins une séquence d'acides nucléiques codant un polypeptide selon l'invention et comprenant en outre un promoteur contrôlant l'expression de ladite au moins une séquence d'acides nucléiques, et , la cellule hôte étant un microorganisme sélectionné parmi les levures.

Dans un mode de réalisation particulier, la molécule d'acides nucléiques isolée comprise dans la cellule hôte comprend en outre un terminateur de transcription de ladite séquence d'acides nucléique, le terminateur de transcription étant de préférence un terminateur de levure.

La cellule hôte est une levure, ladite levure étant en particulier sélectionnée parmi les genres *Saccharomyces, Candida, Ashbya, Dekkera, Pichia (Hansenula), Debaryomyces, Clavispora, Lodderomyces, Yarrowia, Zigosaccharomyces, Schizosaccharomyces, Torulaspora, Kluyveromyces*, *Brettanomycces*, *Cryptococcus* et *Malassezia*.

De manière encore plus particulière, la levure est sélectionnée parmi les espèces *Saccharomyces cerevisiae, Saccharomyces boulardii, Saccharomyces douglasii, Saccharomyces bayanus, Zigosaccharomyces bailii, Schizosaccharomyces pombe, Dekkera brucelensis, Dekkera intermedia, Brettanomycces custersii, Brettanomycces intermedius, Kluyveromyces themotolerens*, *Torulaspora globosa* et *Torulaspora glabrata*.

Encore plus particulièrement, la levure est du genre *Saccharomyces,* de préférence de l'espèce *Saccharomyces cerevisiae.*

Selon un mode de réalisation, la cellule hôte comprend au moins une copie de la molécule d'acides nucléiques telle que définie ci-dessus intégrée dans son génome.

Selon un mode de réalisation, la cellule hôte comprend une copie unique de la molécule d'acides nucléiques telle que définie ci-dessus intégrée dans son génome.

Lorsque la cellule hôte est une cellule de levure, la ou les copies de la molécule d'acides nucléiques peut être intégrée à différents loci, préférentiellement au locus URA3, au locus JLP1, au locus LEU2, ou au locus TRP1 du génome de ladite cellule de levure. Lorsque la cellule hôte est une cellule de levure et que plusieurs copies de la molécule d'acides nucléiques sont intégrées, les différentes copies peuvent être intégrées au même locus, ou encore à différents loci, préférentiellement à l'une quelconque des combinaisons des loci URA3, JLP1, LEU2, et/ou TRP1.

Avantageusement, les codons utilisés dans la molécule d'acides nucléiques ont été adaptés pour une expression optimale dans la cellule hôte sélectionnée.

Une expression optimale peut notamment être obtenue lorsque les codons choisis sont ceux utilisés de manière préférentielle par l'organisme d'origine de la cellule hôte. Les codons utilisés de manière préférentielle sont connus pour la plupart des organismes couramment utilisés dans le domaine. L'homme du métier pourra aisément déterminer les codons les plus avantageux à utiliser en fonction de la cellule hôte choisie.

A cet effet, l'homme du métier sait quelle technique utiliser pour modifier les codons de la molécule d'acide nucléique. Les codons peuvent être par exemple modifiés par mutagénèse dirigée *in vitro* à partir d'un échantillon de la molécule d'acides nucléiques dont les codons sont à adapter, à l'aide d'une amplification par réaction en chaine par polymérase (PCR). Alternativement, la molécule d'acides nucléiques peut être synthétisée *in vitro* directement avec les codons optimisés.

Les cellules hôtes peuvent être cultivées dans des bioréacteurs aérobie ou anaérobie, à petite et grande échelle, dans des flacons ou des boites de Petri. La culture peut être effectuée à une température, un pH, un milieu de culture et une teneur en oxygène appropriés pour une cellule hôte donnée.

### Méthodes

### Méthode pour produire un polypeptide à activité de phloroglucinol synthase

Il est également ici décrit une méthode pour produire un polypeptide à activité de phloroglucinol synthase tel que défini ci-dessus, ladite méthode ne figurant pas dans le texte des revendications.

Selon un mode de réalisation, la méthode pour produire un polypeptide à activité de phloroglucinol synthase tel que défini ci-dessus comprend au moins les étapes consistant en :
(i) l'introduction d'une molécule d'acides nucléiques ou d'un vecteur tels que décrits ci-dessus dans une cellule hôte appropriée conforme à la description qui précède ; et
(ii) la culture *in vitro* de ladite cellule hôte obtenue à l'étape (i) dans des conditions permettant la croissance de ladite cellule hôte et/ou l'expression de ladite molécule d'acides nucléiques, de manière à produire ledit polypeptide.

Selon un autre mode de réalisation, la méthode pour produire un polypeptide à activité de phloroglucinol synthase tel que défini ci-dessus comprend au moins l'étape consistant en :
(i) la culture *in vitro* d'une cellule hôte exprimant ledit polypeptide, par exemple une cellule hôte telle que décrite ci-dessus, dans des conditions permettant la croissance de ladite cellule hôte et/ou l'expression de la molécule d'acides nucléiques contenue dans ladite cellule hôte, de manière à produire ledit polypeptide.

Selon un mode de réalisation possible, la méthode pour produire un polypeptide à activité de phloroglucinol synthase comprend au moins une étape additionnelle choisie parmi les étapes consistant en :
(α) la récupération des cellules exprimant ledit polypeptide, obtenues après l'étape de culture ; et
(B) la purification du polypeptide à partir des cellules récupérées à l'étape (α).

### Méthode pour produire du phloroglucinol

La présente invention concerne également une méthode pour produire du phloroglucinol.

Selon un mode de réalisation M1 qui ne figure pas dans le texte des revendications, la méthode pour produire du phloroglucinol comprend au moins les étapes consistant en :
(i) l'obtention de cellules par la mise en œuvre de l'une des méthodes décrites ci-dessus ;
(ii) la mise en contact des cellules obtenues à l'étape (i) avec un substrat approprié ;
(iii) l'incubation du mélange issu de l'étape (ii) dans des conditions adaptées pour produire du phloroglucinol;
(iv) optionnellement la récupération du milieu réactionnel comprenant le phloroglucinol, obtenu après l'étape (iii) ; et
(v) optionnellement, la purification du phloroglucinol à partir du milieu réactionnel de l'étape (iv).

Selon un autre mode de réalisation M2, la méthode pour produire du phloroglucinol comprend les étapes consistant en :
(i) la mise en contact d'une cellule hôte exprimant le polypeptide à activité de phloroglucinol synthase tel que défini ci-dessus, par exemple une cellule hôte telle que définie ci-dessus, avec un substrat approprié ;
(ii) la culture *in vitro* de la cellule hôte de l'étape (i) dans des conditions permettant la croissance de ladite cellule hôte et/ou l'expression de la molécule d'acides nucléiques contenue dans ladite cellule hôte, de manière à produire du phloroglucinol ;
(iii) optionnellement la récupération du milieu de culture comprenant le phloroglucinol, obtenu après l'étape (ii) ; et
(iv) optionnellement, la purification du phloroglucinol à partir du milieu de culture de l'étape (iii).

Aux fins des méthodes M1 et M2, le substrat est une source de carbone. Avantageusement, la source de carbone est une source de carbone pure ou un coproduit industriel (tel que la mélasse ou les égouts pauvres, par exemple issus de l'industrie sucrière). De préférence, le substrat dans la source de carbone pure ou le coproduit industriel est un sucre simple, tel que le glucose (ou dextrose), le fructose, le galactose, le mannose, le saccharose, le lactose, ou le maltose ; un sucre complexe, tel qu'un monosaccharide, un disaccharide ou trisaccharides, ou encore un polysaccharide comme l'amidon ; un alcool, tel que l'éthanol ; un acide ; un acide gras et leur dérivé d'ester ; ou un mélange de sucres, d'alcools, d'acides et/ou d'acides gras ou leurs dérivés d'ester.

De préférence, le substrat est le glucose. Alternativement, le substrat est l'éthanol.

Selon un autre mode de réalisation M3 qui ne figure pas dans le texte des revendications, la méthode pour produire du phloroglucinol comprend au moins les étapes consistant en :
(i) la mise en contact d'au moins un polypeptide obtenu à l'étape (B) de la méthode telle que décrite ci-dessus, avec un substrat approprié ;
(ii) l'incubation du mélange issu de l'étape (i) dans des conditions adaptées pour produire du phloroglucinol ;
(iii) optionnellement la récupération du milieu réactionnel comprenant le phloroglucinol, obtenu après l'étape (ii) ; et
(iv) optionnellement, la purification du phloroglucinol à partir du milieu réactionnel de l'étape (iii).

Selon un autre mode de réalisation M4, la méthode pour produire du phloroglucinol comprend au moins les étapes consistant en :
(i) la mise en contact d'au moins un polypeptide tel que défini ci-dessus avec un substrat approprié ;
(ii) l'incubation du mélange issu de l'étape (i) dans des conditions adaptées pour produire du phloroglucinol ;
(iii) optionnellement la récupération du milieu réactionnel comprenant le phloroglucinol, obtenu après l'étape (ii) ; et
(iv) optionnellement, la purification du phloroglucinol à partir du milieu réactionnel de l'étape (iii).

Aux fins de ces méthodes M3 et M4, le substrat est un thioester. Avantageusement, le substrat est un acyl-Coenzyme A (ou acyl-CoA) tel que le malonyl-CoA, l'acétyl-CoA, l'hexanoyl-CoA, le decanoyl-CoA, le lauroyl-CoA et le palmitoyl-CoA, ou un mélange de ceux-ci. De préférence, le substrat est le malonyl-CoA.

Selon un mode de réalisation préféré, la purification du phloroglucinol est réalisée par extraction liquide-liquide.

Les exemples qui suivent visent à illustrer la présente invention sans aucune limitation.

Les enzymes respectivement codées par le gène **PHLD** de *Pseudomonas fluorescens* (Zha et al., 2006), par le gène **PKS1** *d'Ectocarpus siliculosus* (Meslet-Cladière et al., 2013) y sont utilisées comme contrôles.

### EXEMPLES

### Exemple 1 : Identification de nouvelles phloroglucinol synthases candidates

Jusqu'à la présente invention, seules deux phloroglucinol synthases avaient été identifiées et caractérisées :
- l'enzyme codée par le gène **PHLD** de *Pseudomonas fluorescens* (Zha et al., 2006), et
- l'enzyme codée par le gène **PKS1** *d'Ectocarpus siliculosus* (Meslet-Cladière et al., 2013).

Les inventeurs ont à présent découvert et caractérisé de nouvelles phloroglucinol synthases en utilisant des analyses génétiques et fonctionnelles.

Comme indiqué dans l'introduction ci-dessus, la caractérisation fonctionnelle exacte d'une polykétide synthase est complexe car cette classe d'enzymes rassemble des protéines présentant de grandes similarités de séquences alors qu'elles peuvent catalyser des réactions sensiblement dissemblables et reconnaître des substrats tout à fait différents.

### 1.1. Sélection de nouvelles enzymes candidates

Afin d'identifier de nouvelles phloroglucinol synthases, les Inventeurs ont identifié des séquences codant pour des polykétide synthases de type III putatives. Les séquences de ces polykétide synthases de type III putatives ainsi identifiées par les Inventeurs ont été analysées et alignées entre elles en utilisant notamment comme base l'alignement des polykétide synthases de type III publié par Meslet-Cladière *et al.* 2013.

L'analyse de cet alignement de séquence a conduit à la sélection d'un groupe d'enzymes candidates, dont les séquences protéiques sont proches de celle du produit du gène PKS1.Es de l'algue *Ectocarpus siliculosus* (Tableaux 1 et 2).

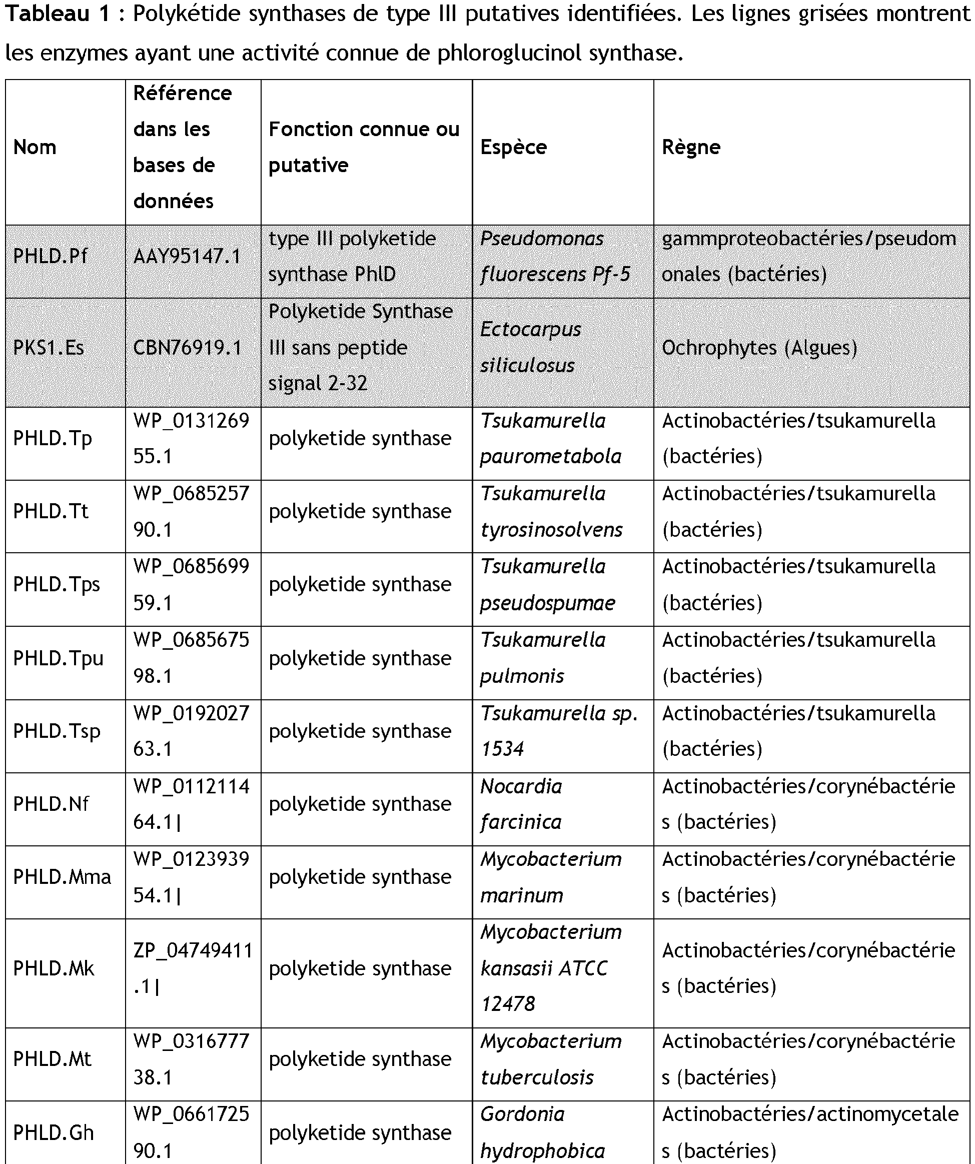

**Tableau 2 : Séquences protéiques des polykétide synthases de type III putatives identifiées.**

| **SEQ ID NO:** | **Nom** | **Séquence protéique** |
|---|---|---|
| SEQ ID NO: 1 | PHLD.Tp | MIAPQIRLGEPDTTPLPDPLWHGAPPAPPTTVAVIESLATGSPAQAHGQSVSAERV AARFADPIQAERIRRVYANTAVATRHLAIDPLSDDFADFSARPDTIRQRMDLYFEHA APLAIETARRALGAVDATEVGQLIFVTSTGFLAPGVDVAVTRSLGLPASTSRVVINFM GCAAAMNALRVASDFVRAHPDRKSLLVCLELSSVNAVFAGDPNDVVISSLFADGCG AALIGASEVGHPLPAGHIVVRDTFAHLLDDAEDGIVLGVNANGITCELAHSLPRYILD |
| | | GVSPVIDGVLARNGLGREDVAHWAIHPGGPKIIESASAALGLPRSASQTSWAVLAE HGNMLSVSLLFVLERLLGARAAGGTQETGLAFSFAPGVTVEGFLFDVVGGPS |
| SEQ ID NO: 2 | PHLD.Tt | MTMNAGSQGAPALDVPAPVLPAPGTWLGAPPAPPTSVAVIESLATGSPAQTYGQ AESADRVAARFDDPRQAERIRRVYAKTRVDERHLAIDPLTPEFAEFSTRPDTVRERM DLFYEHAAPLAVDTARRALGVGTEHEFDPADVGQLVFVTSTGFLAPGVDVAVIRAL GLAPQTSRVVINFMGCAAAMNALRVSTDYVRAHPDRKSLMICLELSSVNAVFSGDP NDVVISSLFADGCGAAVIGASEVGHPLPGGRIVVRDTFTHLLDGAEDGIVLGVNANG ITCELAESLPQYIVDGVAPVIDEVLGRNDLGREAVAHWAIHPGGPKIIESAATALGLP DEASRTSWDVLAEHGNMLSVSLLFVLERLLAQVADGAATQPDGAPTTGMAFSFAP GVTVEGFLFDVVTGDQPGE |
| SEQ ID NO: 3 | PHLD.Tps | MAAPELPGTSVWRGAPPAPPTSVAVIESLATGSPSQAYDQAESADRVASRFDDPR QAERIRRVYAKTRVAERHLAIDPLTPEFAAFSTRPDTIRERMDLFFEHAAPLAIDTARR ALGTVDPADVGQLVFVTSTGFLAPGVDVAVIRALGLSPGTSRVVINFMGCAAAMN ALRVSTDYVRAHPDRKSLMICLELSSVNAVFSGDPNDVVISSLFADGCGAALIGASEV GHPLPAGNIVVRDTFSHLLDGAEDGIVLGVNADGITCELAESLPQYIVDGVAPVVDG VLRRNGLDRDAVAHWAIHPGGPKIIESASTALGLPDEASRLSWDVLAGHGNMLSVS LLFVLERLLAQVASDGADGAPSTGMAFSFAPGVTVEGFLFDVVTTGE |
| SEQ ID NO: 4 | PHLD.Tpu | MNTTEQQSVLPQQAWLGAPPAPPTSVAVIESLATSSPTQTYGQAESADRVAARFD DPRQAERIRRVYAKTRVTERHLAIDPLTPEFAEFSARPDTVRERMDLFFEHAAPLAIE TARRALGDNAATDIGQLVFVTSTGFLAPGVDVAVIRALGLAPQTSRVVINFMGCAA AMNALRVSTDYVRAQPDRKSLMICLELSSVNAVFSGDPNDVVISSLFADGCGAAVI GASEVGHPLPSGQIVVRDTFTHLLDGAEDGIVLGVNANGITCELAESLPQYIVNGVA PVVDAVLDRNGLGREAVAHWAIHPGGPKIIESAAAALGLPDDASRLSWDVLAEHG NMLSVSLLFVLERLRAQVASDGADGAPSTGMAFSFAPGVTVEGFLFDVVTTGE |
| SEQ ID NO: 5 | PHLD.Tsp | MNIQDRSTVAPDSPALGFPPAPPTSVAVIESLATGSPSGVHAQAESADRVASRFDD PAQAERIRRVYTKTRVARRHLAIDPLDEDFAAFSARPDTIRERMDLFAEHASPLAVDT ARRALGAVDPADVGQLVFVTSTGFLAPGVDVAIVRALGLPATTSRVIVNFMGCAAA MNALRVASDFVRAHPDRKSLMVCLELSSVNAVFSGDPNDVVISSLFADGCGAAVIG ASEVGHPLPGGSIVVRDTFAHLLDGAEDGIVLGVNANGITCELAESLPRYIVDGVRPV IDGVLARNGLGHADVAHWAIHPGGPKIIESASAALGLPAAASATSWQVLAEHGNM LSVSLLFVLERMLSGLPDGAPSTGMAFSFAPGVTVEGFLFDIVRDGAPRPGALGGRP A |
| SEQ ID NO: 6 | PHLD.Nf | MSITVDEGGARPATEPRQRIHPDLGHAHTPMPPAPPVTIGVVEGIATGSPAQIVDQ AEAAERVAALFTDPAQRARIARVYEKTRIETRRMAVDPTAPEFRSFSRQPGTLRERM NLFYRHAAPLAVDVAGRALADSGAAAADIGLLVFVTSTGFIAPGVDVAVLRELGLVP TVGRVVVNFMGCAAAMNGIRTGVDYVRAHPDKKALVVCLELSSVNAVFADDVND VIIHSLFGDGCGAVVLGASQARQPLAPGRVVVRDSFSHLFDAAEDGIVLGVDHNGIT CELSENLPRYIYRGVDPVVREVLARNGLRKSDIDLWAIHPGGPKIIEESVRSLGLSPEQ |
| | | AAPSWDVLARHGNMLSVSLIFVLEQMIAQSATAEPISTGVAFSFAPGVTLEGLIFDII RR |
| SEQ ID NO: 7 | PHLD.Mma | MSTAAEGGAIRRAGHEPRYDLAQLPPAPPTTVAVIEGMATGAPQRVVAQADAAA RVSELFVDPQQRERISRIYDKTRIDTRRMAVDPLDDEFDEFRREPATIRDRMNLFYQ HAVPLAVDVAARALDGLPYAPDEIGQLVFVTSTGFIAPGVDVEIVKQLGLPRSISRVV VNFMGCAAAMNAIRTATNYVRAHPSMKALVVCIELSSVNAVFADDINDVVIHSLFG DGCGALVIGASQVQQPLPAGNVVIRSSFSQLLDDSEDGIVLGVNHDGITCELSENLP SYIYRSVDPVVAEMLRDNGLSKADIDLWAIHPGGPKIIEQSARSLGIPVGRAVQSWD VLAQFGNMLSVSLIFVLEMMVAQAESDKPISTGVAFAFAPGVTVEGMLFDIIRR |
| SEQ ID NO: 8 | PHLD.Mk | MSSAADGGAPVADVPGYEPHYDLAQLPPAPPTTVAVIEGMATGVPQRVVRQSDA AARVAQMFVDPQQRERVSRVYAKTRIDTRRMAVNPLDAEFDAFRREPATIRDRMS LFYRHAVPLAVEVTRRALAGLSYGADEIGLLVFVTSTGFVAPGVDVAIVKELGLSRAIS RVVVNFMGCAAAMNAIRTATNYVRAHPAMKALVVCIELCSVNAVFADNVKDVVI HSLFGDGCAALVIGASQVQQQLPAGSVVIRSNFSQLLDDAEDGIVLGVNHDGITCEL SENLPDYIYRGVAPVVANVLYDNGLQQSDIDLWAIHPGGPKIIEQSVRSLGIGVECAA PSWDVLARYGNMLSVSLIFVLEMMVQQAESEKPLSTGVAFAFAPGVTVEGMLFDI VRR |
| SEQ ID NO: 9 | PHLD.Mt | MNVSAESGAPRRAGQRHEVGLAQLPPAPPTTVAVIEGLATGTPRRVVNQSDAADR VAELFLDPGQRERIPRVYQKSRITTRRMAVDPLDAKFDVFRREPATIRDRMHLFYEH AVPLAVDVSKRALAGLPYRAAEIGLLVLATSTGFIAPGVDVAIVKELGLSPSFGDGCA ALVIGASQVQEKLEPGKVVVRSSFSQLLDNTEDGIVLGVNHNGITCELSENLPGYIFS GVAPVVTEMLWDNGLQISDIDLWAIHPGGPKIIEQSVRSLGISAELAAQSWDVLAR FGNMLSVSLIFVLETMVQQAESAKAISTGVAFAFGPGVTVEGMLFDIIRR |
| SEQ ID NO: 10 | PHLD.Gh | MPAPVTTVAVIEAVATGAPATVHPQTRAAEQVAELYDDPALQERIRRLYRNTRVQT RHLAVDPMTPEFQEFSSRPATVRTRMNDYFHHAVPLAVDVARRALAGVTDPATEI GQIIFVTSTGFIAPGVDVAVITELGLAPTVHRVIINFMGCAAAINGISTATDHVRANP DSRALLICLELSSVNAVFGADPVELVTHSLFGDGCGAMLIGASPVGRRLAPGQLVVR DTFSHLFHDTGDGIVLGVNDDGITCELAQELPSYIRRGVGPAIDAALNRSRLRRDDIA HWAIHPGGPAIIEQSVAALDLPPDRAATSWEVLAEYGNMLSVSLVFVLEKLIAAGAH GRGQAPETGVAFSFAPGVALEGMLFDLVC |

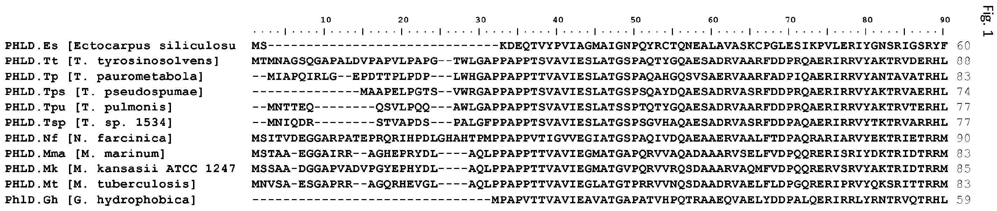

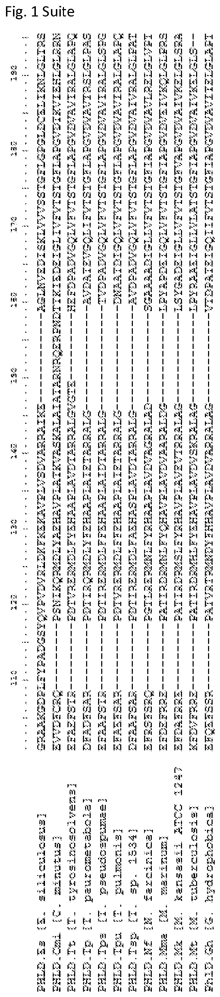

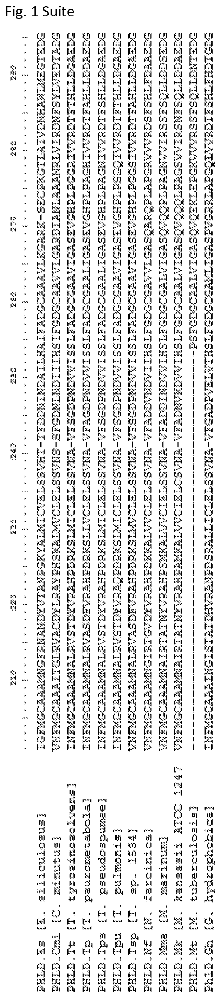

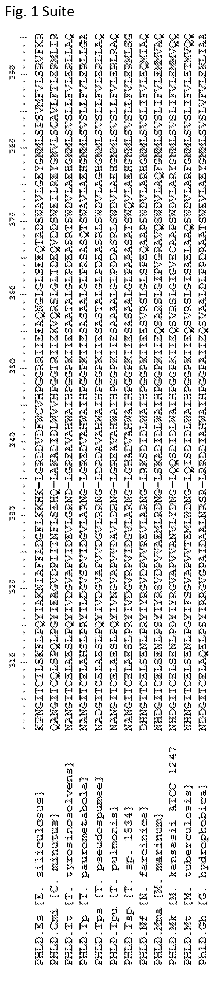

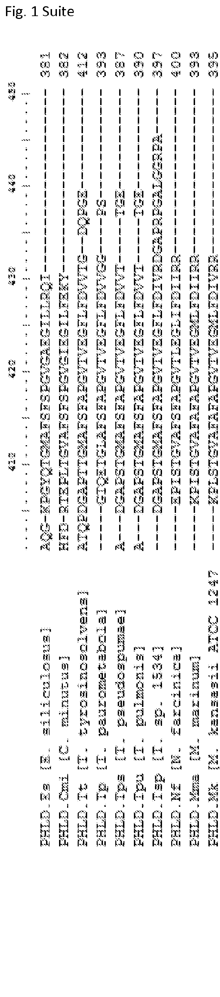

La Figure 1 présente l'alignement des séquences protéiques des enzymes candidates listées dans le Tableau 1. L'alignement des dix enzymes candidates sélectionnées avec l'enzyme PKS1.Es a été réalisé en employant le logiciel Clustal W.

Le Tableau 3 présente la matrice des identités de séquence existant entre ces différentes enzymes candidates et PHLD.Pf et PKS1.Es.

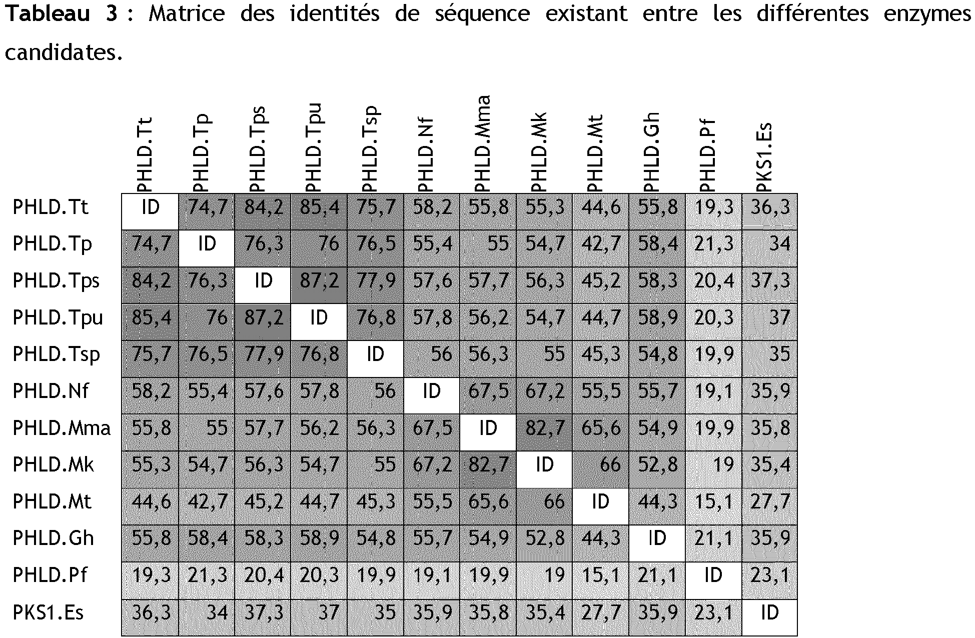

Les résultats montrent que la distance phylogénétique existant entre les enzymes candidates et l'enzyme bactérienne PHLD.Pf est importante puisque moins de 25 % d'identité sont observés entre ces deux groupes. Il n'est donc pas possible d'affecter *a priori* une fonction phloroglucinol synthase aux polykétide synthases putatives étudiées à cause de cette forte divergence de séquences.

### 1.2. Mesure des activités phloroglucinol synthases chez la levure

Afin d'identifier les phloroglucinol synthases parmi les candidats identifiés ci-dessus, une méthode d'extraction et de dosage du phloroglucinol a été mise au point comme détaillé ci-dessous.

### 1.2.1. Méthode d'extraction du phloroglucinol

La méthode a été développée en employant le résorcinol comme étalon interne. Différents tests ont conduit au développement d'une méthode d'extraction liquide-liquide réalisée à pH 4.0 en présence d'acétate d'éthyle comme solvant, et en saturant la phase aqueuse en NaCl. L'extraction est réalisée pendant 30 min sous agitation circulaire. La phase organique est prélevée et le solvant acétate d'éthyle est évaporé sous un flux d'azote N₂ à 30°C. L'extrait sec obtenu après évaporation complète est alors repris dans un volume déterminé d'un mélange 50 % -50 % éthanol/H₂0.

Le rendement d'extraction a été mesuré par spectroscopie de masse après chromatographie haute pression sur une colonne C18 (dimensions : 100mm*2.1 mm ; granulométrie : 1.7 µm) en employant un gradient 0.03% acide méthanoïque (HCOOH)/ acétonitrile (ACN).

Les rendements d'extraction ont été déterminés en utilisant des solutions de phloroglucinol et résorcinol préparées dans le milieu de culture utilisé pour la croissance des levures. Les concentrations de phloroglucinol correspondent aux points bas (20 µg.mL⁻¹) et haut (200 µg.mL⁻¹) de la gamme de dosage. La concentration du résorcinol correspond à la concentration ajoutée en tant qu'étalon interne lors des dosages (200 µg.mL⁻¹). Les résultats sont présentés dans le Tableau 4.

**Tableau 4 : Rendement d'extraction du phloroglucinol (20 et 200 µg.mL⁻¹) et du résorcinol (200 µg.mL⁻¹), extraits par l'acétate d'éthyle, selon la méthode décrite.**

| **Produit** | **Phloroglucinol** | | **Résorcinol (EI)** |
|---|---|---|---|
| **Concentration de phloroglucinol ou de résorcinol dans le milieu de culture (µg.mL⁻¹)** | 20 | 200 | 200 |
| **RDT (%)** | 76 | 89 | 82 |

### 1.2.2. Développement d'une méthode d'analyse UPLC/UV et UPLC/MS

Une méthode d'analyse par chromatographie UPLC et mesure d'absorbance en UV (rayonnement ultraviolet) a été développée. L'extrait est chromatographié sur une colonne propyl-pentafluorophenyl (PFP) de dimensions 100mm*2.1 mm ; granulométrie :1.8 µm selon un gradient de 0.1% HCOOH/ACN - 0.1% HCOOH. Le phloroglucinol est détecté en UV à 230 nm. Une méthode UPLC couplée à un spectromètre de masse (UPLC/Mass) a également été développée.

La quantification est réalisée en employant une gamme de 20 à 200 µg.ml⁻¹ de phloroglucinol dilué dans du milieu de culture de levure (Yeast Extract 1%, BactoPeptone 2 %) en présence d'une quantité fixe de résorcinol, employé comme témoin interne. La quantité de phloroglucinol est déterminée en calculant les rapports de surface des pics de chromatographie phloroglucinol / résorcinol.

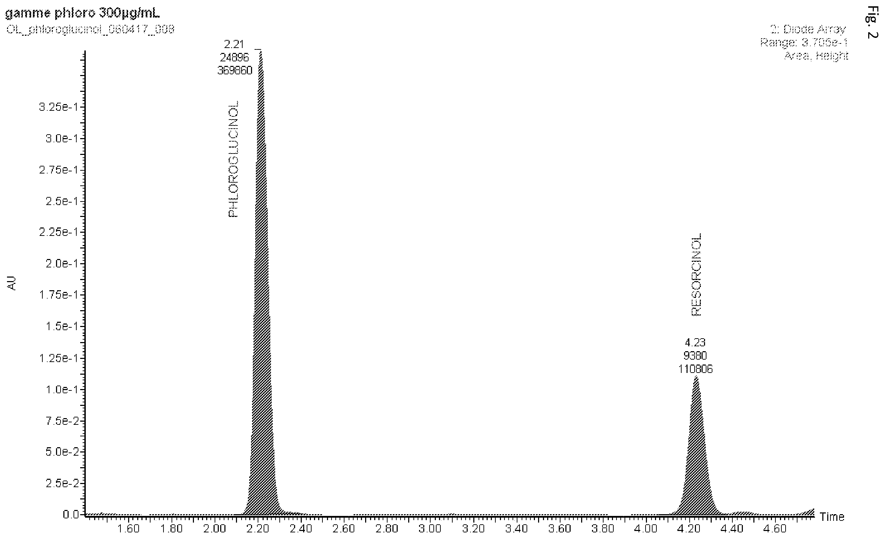

La Figure 2 présente un exemple de pics de chromatographie de phloroglucinol / résorcinol obtenus sur colonne PFP.

Cette méthode de dosage permet donc de mesurer qualitativement et quantitativement, de manière fiable, le phloroglucinol présent dans un échantillon.

La méthode UPLC couplée à un spectromètre de masse (UPLC/mass) permet de doser les échantillons contenant une concentration de phloroglucinol allant de 2 à 50 µg.mL⁻¹.

### Exemple 2 : Identification de nouvelles phloroglucinol synthases fonctionnelles

### 2.1. Expression des gènes candidats dans la levure Saccharomyces cerevisiae sous forme multicopies

Les 10 gènes candidats (PHLD.Tp, PHLD.Tt, PHLD.Tps, PHLD.Tpu, PHLD.Tsp, PHLD.Nf, PHLD.Mma, PHLD.Mk, PHLD.Mt et PHLD.Gh) sélectionnés et décrits ci-dessus, ainsi que les gènes PHLD.Pf et PKS1.Es codant les deux phloroglucinol synthases identifiées à ce jour, utilisées comme contrôles (voir Tableaux 1 et 2), ont été synthétisés en adaptant les codons utilisés pour une expression optimale dans la levure *S*. *cerevisiae.*

Cette adaptation des codons a été réalisée afin d'optimiser l'expression de ces différents gènes dans les cellules de levure (ces 12 gènes synthétiques codent des protéines strictement identiques aux protéines exprimées par les organismes d'origine). Chacun de ces gènes a été placé sous le contrôle du même promoteur de levure ADH2 (pADH2) qui permet leur expression notamment lorsque le milieu de culture contient de l'éthanol comme source de carbone. Le terminateur de transcription du gène de levure RPL3 (tRPL3) a été placé en aval de chacun des 12 gènes placés sous le contrôle du promoteur ADH2.

La Figure 3 montre un exemple d'unité génique ainsi construite pour un candidat ou un contrôle donné (PHLD.ii).

Les différentes unités géniques ainsi construites ont été intégrées de manière indépendante au locus URA3 du génome d'une souche sauvage de la levure *S*. *cerevisiae.* La souche de type sauvage utilisée est la souche commerciale W303 (génotype : MAT-a, his3, leu2, trp1, ura3, ade-). La technique d'intégration employée permet l'intégration d'un nombre variable de copies de chaque unité génique. Pour chaque construction, le nombre de copies d'unités géniques intégrées a été déterminé par PCR quantitative selon la méthode classique Taqman. Les souches de levure exprimant différents nombres de copies de chacun des 10 gènes candidats PHLD.ii ou des gènes contrôles PHLD.Pf et PKS1.Es décrits ci-dessus, ont été cultivées en présence de 20 g.L⁻¹ d'éthanol comme source de carbone pendant 48 heures à 30 °C. Les 12 souches de levures obtenues de manière indépendante après transformation et intégration au locus *URA3* des unités géniques décrites ci-dessus ont ainsi été analysées pour leur capacité à produire du phloroglucinol. La souche parentale sauvage W303 a été cultivée dans les mêmes conditions et utilisée à titre de témoin.

Les densités optiques (DO) des différentes cultures ont été mesurées à 600 nm (DO₆₀₀), indiquant ainsi le niveau de croissance de chaque souche (Figure 4A et B) .

La capacité des différentes souches de levures exprimant les différents gènes PHLD.ii sous le contrôle du promoteur ADH2 à synthétiser du phloroglucinol a été testée en utilisant la méthode d'extraction et de dosage mise au point telle que décrite au paragraphe 1.2 ci-dessus. Le niveau de production de phloroglucinol (en mg.L⁻¹) a été mesuré dans le milieu de culture (Figures 4A et C).

La Figure 4 montre que l'ensemble des souches exprimant des nombres différents de copies des gènes PHLD.Tp, PHLD.Tt, PHLD.Tps, PHLD.Tpu, PHLD.Tsp, PHLD.Nf, PHLD.Mma, PHLD.Mk, PHLD.Mt et PHLD.Gh produit une quantité significative de phloroglucinol qui est excrété dans le milieu de culture (Fig. 4A, C et D). Ces résultats indiquent donc que chacun de ces 10 gènes exprime une phloroglucinol synthase active chez la levure.

De plus, comme attendu, aucune production de phloroglucinol n'a été mesurée dans la souche parentale contrôle W303 (données non montrées).

Ainsi, cette étude fonctionnelle a permis d'identifier dix nouvelles phloroglucinol synthases, codées respectivement par les gènes PHLD.Tp, PHLD.Tt, PHLD.Tps, PHLD.Tpu, PHLD.Tsp, PHLD.Nf, PHLD.Mma, PHLD.Mk, PHLD.Mt et PHLD.Gh. Cette étude révèle pour la première fois que des bactéries Gram+, en particulier des bactéries actinomycètes, codent des phloroglucinol synthases fonctionnelles (PHLD.Tp, PHLD.Tt, PHLD.Tps, PHLD.Tpu, PHLD.Tsp, PHLD.Nf, PHLD.Mma, PHLD.Mk, PHLD.Mt et PHLD.Gh).

Cette étude révèle également pour la première fois que l'enzyme PKS1.ES est fonctionnelle lorsqu'elle est exprimée chez un système eucaryote hétérologue, la levure. Cette étude révèle en outre de manière inattendue que PHLD.Pf ne code pas une phloroglucinol synthase fonctionnelles chez la levure. Ce résultat est surprenant car l'enzyme codée par PHLD.Pf présente une activité phloroglucinol synthase démontrée lorsqu'elle est exprimée chez *Escherichia coli* (Achkar et al., 2005).

### 2.2. Expression des gènes candidats dans la levure Saccharomyces cerevisiae sous forme d'une copie unique

La production de phloroglucinol par des souches n'ayant intégré qu'une seule copie du gène candidat PHLD sélectionnés et identifiées comme étant fonctionnels chez la levure (voir les résultats du paragraphe précédent et la Fig. 4) a également été évaluée. Le gène PKS1.Es codant la phloroglucinol synthase fonctionnelle chez la levure selon les résultats décrits au paragraphe 2.1 ci-dessus a été utilisé comme contrôle positif. Le gène PHLD.Pf codant la phloroglucinol synthase non fonctionnelle chez la levure selon les résultats décrits au paragraphe 2.1 ci-dessus a été utilisé comme contrôle négatif.

Chacun de ces gènes a été placé soit sous le contrôle du promoteur de levure ADH2 (pADH2), qui permet leur expression notamment lorsque le milieu de culture contient de l'éthanol comme source de carbone, soit sous contrôle du promoteur de levure CCW12 (pCCW12), qui permet leur expression, notamment lors de la glycolyse, lorsque le milieu de culture contient du glucose comme source de carbone. Le terminateur de transcription du gène de levure RPL3 (tRPL3) a été placé en aval de chacune des constructions.

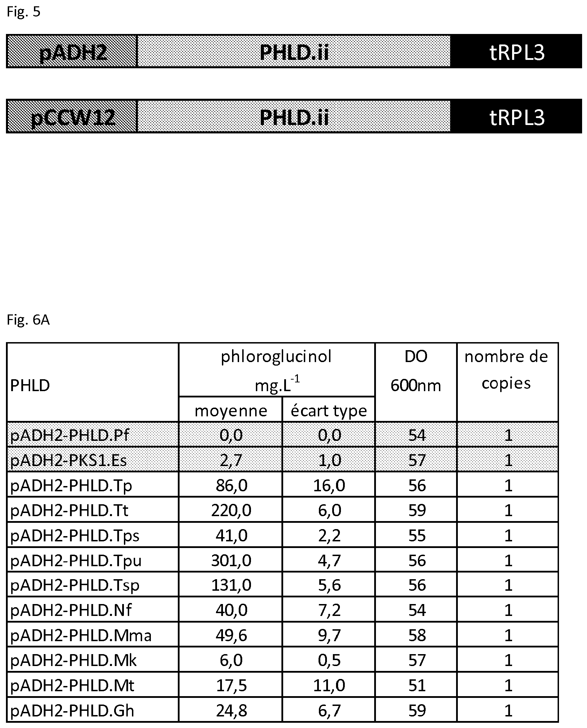

Le détail des différentes constructions géniques réalisées est reporté en Figure 5.

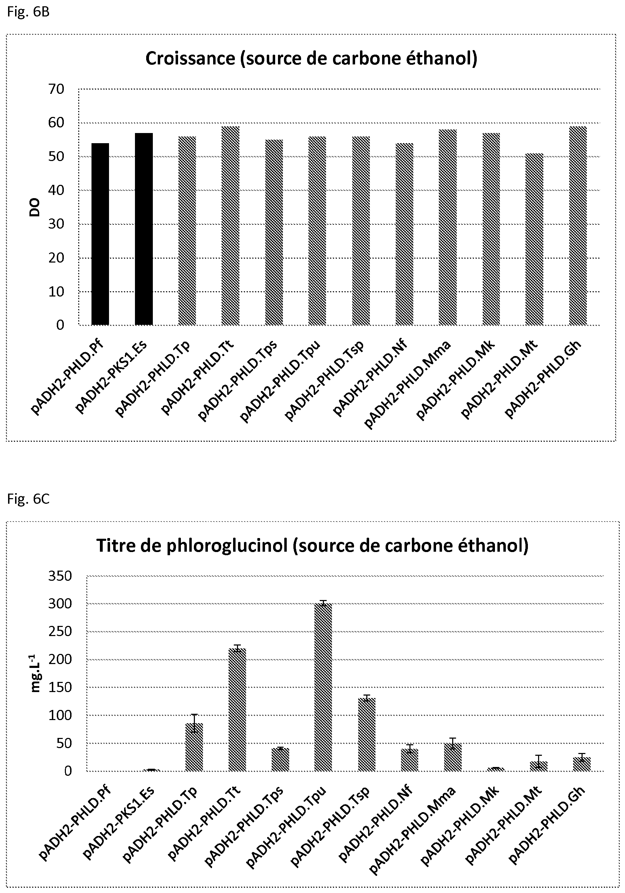

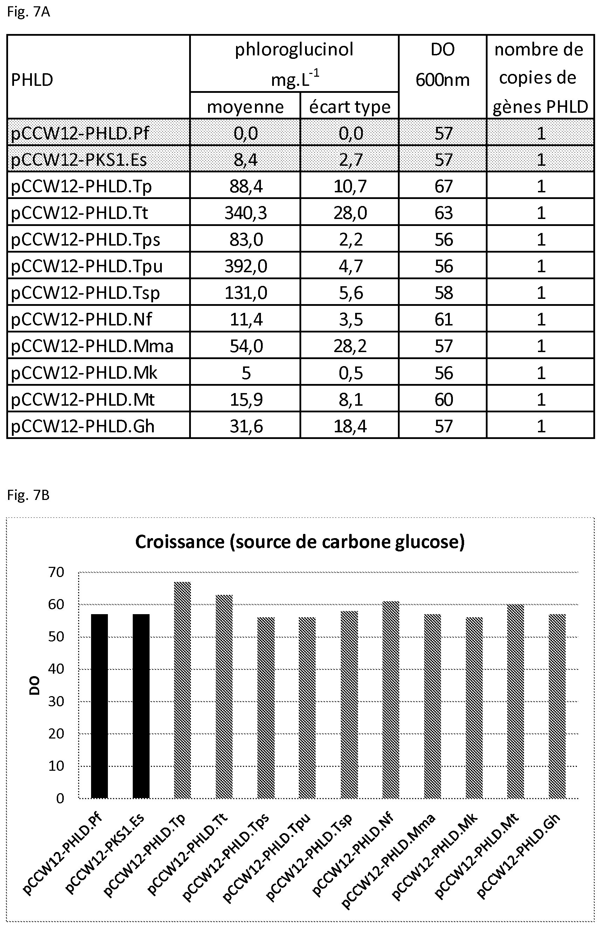

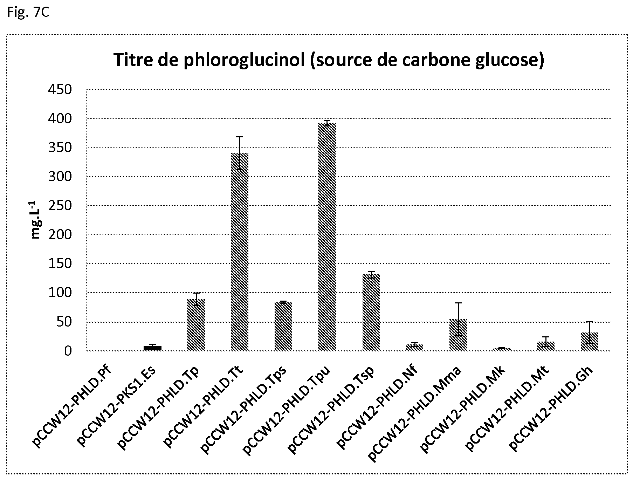

Les souches de levure exprimant une copie unique de PHLD ou PKS1 ont été cultivées en présence de 20 g.L⁻¹ d'éthanol comme source de carbone (constructions contrôlées par le pADH2) ou en présence de 20g.L⁻¹ de glucose (constructions contrôlées par le pCCW12) pendant 48 heures à 30 °C (Figures 6 et 7). Les souches ont été cultivées et analysées pour leur capacité à produire du phloroglucinol.

Les densités optiques des différentes cultures ont été mesurées à 600 nm (indiquant le niveau de croissance de chaque souche, Figures 6A et B et Figures 7A et B) et la production de phloroglucinol (en mg.L⁻¹) a été mesurée dans le milieu de culture, pour 2 transformants indépendants pour chaque construction (Figures 6A et C et Figures 7A et 9C).

La Figure 6 (A et C) montre que les souches exprimant une seule copie des gènes PHLD.Tp, PHLD.Tt, PHLD.Tps, PHLD.Tpu, PHLD.Tsp, PHLD.Nf, PHLD.Mma, PHLD.Mk, PHLD.Mt ou PHLD.Gh sous le contrôle du promoteur ADH2 synthétisent une quantité mesurable et significative de phloroglucinol qui est excrété dans le milieu de culture, en présence d'éthanol comme source de carbone.

Pour les cultures réalisées en présence de glucose comme source de carbone, les souches exprimant une seule copie des gènes PHLD.Tp, PHLD.Tt, PHLD.Tps, PHLD.Tpu, PHLD.Tsp, PHLD.Nf, PHLD.Mma, PHLD.Mk, PHLD.Mt et PHLD.Gh sous le contrôle du promoteur CCW12 synthétisent toutes du phloroglucinol, en quantité comparable aux cultures réalisées en présence d'éthanol comme source de carbone (Figures 7A et C).

Les résultats obtenus ci-dessus confirment les résultats obtenus dans les souches contenant plusieurs copies des phloroglucinol synthases décrits dans le paragraphe 2.1 ci-dessus. Ainsi, l'expression des enzymes candidates PHLD.Tp, PHLD.Tt, PHLD.Tps, PHLD.Tpu, PHLD.Tsp, PHLD.Nf, PHLD.Mma, PHLD.Mk, PHLD.Mt et PHLD.Gh conduit à la production significative de phloroglucinol par les cellules de levure. Ces résultats montrent que ces gènes codent des phloroglucinol synthases et que ces phloroglucinol synthases sont actives dans des cellules de levure.

Ces résultats montrent en outre que les niveaux de production de phloroglucinol sont élevés même lorsqu'une seule copie du gène codant la phloroglucinol synthase est exprimée.

Ces résultats sont les premières démonstrations de l'existence d'une activité phloroglucinol synthase chez les bactéries de type gram+.

De manière plus générale, les résultats montrent que les enzymes candidates PHLD.ii testées présentent une activité globalement plus élevée que les deux seules phloroglucinol synthases connues avant cette étude, i.e. les enzymes d'*E. siliculosus* et *P. fluorescens*. Les enzymes candidates PHLD du genre *Tsukamurella* (PHLD.Tt, PHLD.Tps, PHLD.Tpu, PHLD.Tsp, PHLD.Tp) sont particulièrement actives lorsqu'elles sont exprimées chez la levure. En effet, la production de phloroglucinol normalisée par le nombre de copies démontre que les enzymes de *Tsukamurella* testées (PHLD.Tp, PHLD.Tt, PHLD.Tpu, PHLD.Tps et PHLD.Tsp) ont une activité phloroglucinol synthase au moins 10 fois plus importante que celle de l'enzyme PKS1 *d'Ectocarpus siliculosus* dans le système d'expression en levure testé ici. Selon ces résultats, l'enzyme PHLD.Tpu serait notamment l'enzyme la plus active, dans les différentes conditions testées.

Les 3 enzymes **PHLD.Mma** (de *Mycobacterium marinum),* **PHLD.Nf** (de *Nocardia farcinica)* et **PHLD.Gh** (de *Gordonia hydrophobica*) sont également significativement très actives.

La présente invention fournit donc plusieurs nouvelles voies originales et avantageuses de biosynthèse du phloroglucinol chez la levure.

### 2.3. Observations complémentaires

L'étude réalisée par les Inventeurs et rapportée ici a permis d'identifier **10 nouvelles enzymes** présentant une activité phloroglucinol synthase. Ces 10 nouvelles phloroglucinol synthase proviennent de bactéries gram+ de l'ordre des *Actinomycétales* dont le genre *Tsukamurella* (PHLD.Tt, PHLD.Tps, PHLD.Tpu, PHLD.Tsp, PHLD.Tp), le genre *Nocardia* (PHLD.Nf) ; le genre *Mycobacterium* (PHLD.Mma, PHLD.Mt et PHLD.Mk) et le genre *Gordonia* (PHLD.Gh) et n'ont jamais été décrites jusque-là comme produisant du phloroglucinol.

Ces résultats démontrent donc pour la première fois et de manière univoque qu'il est possible de synthétiser du phloroglucinol en cellules de levure.

Il est important de noter que le phloroglucinol synthétisé est sécrété à plus de 95 % dans le milieu de culture par les cellules de levure. Cette sécrétion particulièrement efficace est très favorable à la mise en œuvre d'un procédé de bio-production du phloroglucinol.

Les phloroglucinol synthases des bactéries actinomycètes du genre *Tsukamurella* seraient les plus actives d'après les résultats obtenus à ce jour. En effet, les résultats obtenus montrent que les enzymes PHLD de *Tsukamurella sp*., exprimées au sein de cellules de levure, sont au moins 10 fois plus actives que la phloroglucinol synthase de l'algue brune *Ectocarpus siliculosus* de l'art antérieur, dans les conditions testées.

Les enzymes PHLD de *Tsukamurella sp.* pourraient donc représenter des enzymes de choix pour mettre en œuvre un procédé de bio-production du phloroglucinol chez la levure *S*. *cerevisiae.* Enfin, les enzymes identifiées par les Inventeurs sont originales en termes d'espèces d'origine et en termes de séquences protéiques. En effet, il est montré pour la première fois que des bactéries Gram+ codent des phloroglucinol synthase fonctionnelles. En outre, la séquence protéique de l'enzyme la plus active dans les conditions expérimentales testées par les Inventeurs, PHLD.Tpu de *Tsukamurella pulmonis*, diffère de manière significative de la séquence de l'enzyme connue PKS1.Es *d'Ectocarpus siliculosus* (différence de plus de 63 %, Tableau 3).

### REFERENCES BIBLIOGRAPHIQUES

Achkar J et al., (2005) « Biosynthesis of phloroglucinol » J Am Chem Soc. 127:5332-5333*.*
Meslet-Cladière L, Delage L, Leroux CJ, Goulitquer S, Leblanc C, Creis E, Gall EA, Stiger-Pouvreau V, Czjzek M, and Potin P. (2013) "Structure/function analysis of a type III polykétide synthase in the brown alga Ectocarpus siliculosus reveals a biochemical pathway in phlorotannin monomer biosynthesis. " Plant Cell. 25:3089-3103*.*
Zha W, Rubin-Pitel SB and Zhao H. (2006) "Characterization of the substrate specificity of PHLD, a type III polykétide synthase from Pseudomonas fluorescens. " J Biol Chem. 281:32036-32047*.*

## Revendications

1. Utilisation d'au moins un polypeptide choisi parmi les polyketide synthases de type III de bactéries actinomycètes, comme phloroglucinol synthase ; ledit polypeptide comprenant au moins une séquence d'acides aminés ayant au moins 60% d'identité avec une séquence choisie parmi SEQ ID NO: 4, SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 9 et SEQ ID NO: 10.

2. Utilisation selon la revendication 1, ledit polypeptide comprenant au moins une séquence d'acides aminés ayant au moins 65% d'identité avec une séquence choisie parmi SEQ ID NO: 4, SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 9 et SEQ ID NO: 10.

3. Utilisation selon la revendication 1 ou 2, lesdites bactéries actinomycètes étant choisies dans le groupe constitué par *Tsukamurella sp*., *Nocardia sp*., *Mycobacterium sp.* et *Gordonia sp*., en particulier *Tsukamurella pulmonis*, *Tsukamurella paurometabola Tsukamurella tyrosinosolvens, Tsukamurella pseudospumae, Tsukamurella sp. 1534, Nocardia farcinica, Mycobacterium marinum*, *Mycobacterium kansasii*, *Mycobacterium tuberculosis* et *Gordonia hydrophobica*.

4. Utilisation selon l'une quelconque des revendications 1 à 3, ledit polypeptide comprenant au moins une séquence d'acides aminés ayant au moins 70% d'identité, de préférence encore au moins 75% d'identité, de manière encore préférée au moins 80% d'identité, de manière encore plus préférée au moins 85% d'identité, de manière toujours plus préférée au moins 90% d'identité, de manière plus préférentielle encore au moins 95% d'identité, de manière encore préférée au moins 96% d'identité, de manière encore plus préférée au moins 97% d'identité, de manière toujours plus préférée au moins 98% d'identité, et de manière plus préférentielle encore au moins 99% d'identité, avec une séquence choisie parmi SEQ ID NO: 4, SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 9 et SEQ ID NO: 10.

5. Utilisation selon l'une quelconque des revendications 1 à 4, ledit polypeptide comprenant au moins une séquence d'acides aminés choisie parmi SEQ ID NO: 4, SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 9 et SEQ ID NO: 10.

6. Cellule hôte comprenant au moins une molécule d'acides nucléiques isolée ou au moins un vecteur comprenant au moins une molécule d'acides nucléiques isolée, la molécule d'acides nucléiques isolée comprenant au moins une séquence d'acides nucléiques codant un polypeptide tel que défini dans l'une quelconque des revendications 1 à 5 et comprenant en outre un promoteur contrôlant l'expression de ladite au moins une séquence d'acides nucléiques, et la cellule hôte étant un microorganisme sélectionné parmi les levures.

7. Cellule hôte selon la revendication 6, le promoteur étant un promoteur de levure.

8. Cellule hôte comprenant au moins une molécule d'acides nucléiques isolée ou au moins un vecteur comprenant au moins une molécule d'acides nucléiques isolée, la molécule d'acides nucléiques isolée comprenant au moins une séquence d'acides nucléiques codant un polypeptide tel que défini dans l'une quelconque des revendications 1 à 5 et comprenant en outre un terminateur contrôlant l'expression de ladite au moins une séquence d'acides nucléiques, et la cellule hôte étant un microorganisme sélectionné parmi les levures.

9. Cellule hôte selon la revendication 6 ou 7, la molécule d'acides nucléiques isolée comprenant en outre un terminateur de transcription de ladite séquence d'acides nucléiques.

10. Cellule hôte selon la revendication 9, le terminateur de transcription étant un terminateur de levure.

11. Cellule hôte selon l'une quelconque des revendications 6 à 10 , ladite levure étant en particulier sélectionnée parmi les genres *Saccharomyces*, *Candida*, *Ashbya*, *Dekkera*, *Pichia (Hansenula), Debaryomyces, Clavispora, Lodderomyces, Yarrowia, Zigosaccharomyces, Schizosaccharomyces*, *Torulaspora*, *Kluyveromyces*, *Brettanomycces*, *Cryptococcus* et *Malassezia* ; de manière plus particulière parmi les espèces *Saccharomyces cerevisiae*, *Saccharomyces boulardii, Saccharomyces douglasii, Saccharomyces bayanus, Zigosaccharomyces bailii, Schizosaccharomyces pombe, Dekkera brucelensis, Dekkera intermedia, Brettanomycces custersii, Brettanomycces intermedius, Kluyveromyces themotolerens, Torulaspora globosa et Torulaspora glabrata.*

12. Cellule hôte selon la revendication 11, la levure étant du genre *Saccharomyces.*

13. Cellule hôte selon la revendication 12, la levure étant de l'espèce *Saccharomyces cerevisiae.*

14. Méthode pour produire du phloroglucinol, comprenant les étapes consistant en :
(i) la mise en contact d'une cellule hôte exprimant le polypeptide à activité de phloroglucinol synthase tel que défini dans l'une quelconque des revendications 1 à 5, par exemple une cellule hôte selon l'une quelconque des revendications 6 à 13, avec un substrat approprié ;
(ii) la culture *in vitro* de la cellule hôte de l'étape (i) dans des conditions permettant la croissance de ladite cellule hôte et/ou l'expression de la molécule d'acides nucléiques contenue dans ladite cellule hôte, de manière à produire du phloroglucinol ;
(iii) optionnellement la récupération du milieu de culture comprenant le phloroglucinol, obtenu après l'étape (ii) ; et
(iv) optionnellement, la purification du phloroglucinol à partir du milieu de culture de l'étape (iii).

15. Méthode pour produire du phloroglucinol, comprenant au moins les étapes consistant en :
(i) la mise en contact d'au moins un polypeptide tel que défini dans l'une quelconque des revendications 1 à 5 avec un substrat approprié ;
(ii) l'incubation du mélange issu de l'étape (i) dans des conditions adaptées pour produire du phloroglucinol ;
(iii) optionnellement la récupération du milieu réactionnel comprenant le phloroglucinol, obtenu après l'étape (ii) ; et
(iv) optionnellement, la purification du phloroglucinol à partir du milieu réactionnel de l'étape (iii).

## Patentansprüche

1. Verwendung von mindestens einem Polypeptid, das aus Polyketidsynthasen vom Typ III von Aktinomyzeten, beispielsweise Phloroglucinolsynthase, ausgewählt ist; wobei das Polypeptid mindestens eine Aminosäuresequenz umfasst, die eine Übereinstimmung von mindestens 60% mit einer Sequenz aufweist, die ausgewählt ist aus SEQ ID NO 4, SEQ ID NO 1, SEQ ID NO 2, SEQ ID NO 3, SEQ ID NO 5, SEQ ID NO 6, SEQ ID NO 7, SEQ ID NO 8, SEQ ID NO 9 und SEQ ID NO 10.

2. Verwendung nach Anspruch 1, wobei das Polypeptid mindestens eine Aminosäuresequenz umfasst, die eine Übereinstimmung von mindestens 65% mit einer Sequenz aufweist, die ausgewählt ist aus SEQ ID NO 4, SEQ ID NO 1, SEQ ID NO 2, SEQ ID NO 3, SEQ ID NO 5, SEQ ID NO 6, SEQ ID NO 7, SEQ ID NO 8, SEQ ID NO 9 und SEQ ID NO 10.

3. Verwendung nach Anspruch 1 oder 2, wobei die Aktinomyzeten aus der Gruppe ausgewählt sind, die aus Tsukamurella sp., Nocardia sp., Mycobacterium sp. und Gordonia sp. besteht, insbesondere Tsukamurella pulmonis, Tsukamurella paurometabola, Tsukamurella tyrosinosolvens, Tsukamurella pseudospumae, Tsukamurella sp. 1534, Nocardia farcinica, Mycobacterium marinum, Mycobacterium kansasii, Mycobacterium tuberculosis und Gordonia hydrophobica.

4. Verwendung nach einem der Ansprüche 1 bis 3, wobei das Polypeptid mindestens eine Aminosäuresequenz umfasst, die eine Übereinstimmung von mindestens 70%, noch mehr bevorzugt von mindestens 75%, auf weiter bevorzugte Weise von mindestens 80%, auf noch stärker bevorzugte Weise von mindestens 85%, auf wiederum stärker bevorzugte Weise von mindestens 90%, auf noch stärker bevorzugte Weise von mindestens 95%, auf weiter bevorzugte Weise von mindestens 96%, auf noch stärker bevorzugte Weise von mindestens 97%, auf wiederum stärker bevorzugte Weise von mindestens 98% und auf noch stärker bevorzugte Weise von mindestens 99% mit einer Sequenz aufweist, die ausgewählt ist aus SEQ ID NO 4, SEQ ID NO 1, SEQ ID NO 2, SEQ ID NO 3, SEQ ID NO 5, SEQ ID NO 6, SEQ ID NO 7, SEQ ID NO 8, SEQ ID NO 9 und SEQ ID NO 10.

5. Verwendung nach einem der Ansprüche 1 bis 4, wobei das Polypeptid mindestens eine Aminosäuresequenz umfasst, die ausgewählt ist aus SEQ ID NO 4, SEQ ID NO 1, SEQ ID NO 2, SEQ ID NO 3, SEQ ID NO 5, SEQ ID NO 6, SEQ ID NO 7, SEQ ID NO 8, SEQ ID NO 9 und SEQ ID NO 10.

6. Wirtszelle, die mindestens ein isoliertes Nukleinsäuremolekül oder mindestens einen Vektor umfasst, der mindestens ein isoliertes Nukleinsäuremolekül umfasst, wobei das isolierte Nukleinsäuremolekül mindestens eine Aminosäuresequenz umfasst, die für ein Polypeptid gemäß der Begriffsbestimmung in einem der Ansprüche 1 bis 5 codiert und ferner einen Promotor umfasst, der die Expression der mindestens einen Nukleinsäuresequenz steuert, und wobei die Wirtszelle ein Mikroorganismus ist, der aus Hefen ausgewählt ist.

7. Wirtszelle nach Anspruch 6, wobei der Promotor ein Hefepromotor ist.

8. Wirtszelle, die mindestens ein isoliertes Nukleinsäuremolekül oder mindestens einen Vektor umfasst, der mindestens ein isoliertes Nukleinsäuremolekül umfasst, wobei das isolierte Nukleinsäuremolekül mindestens eine Aminosäuresequenz umfasst, die für ein Polypeptid gemäß der Begriffsbestimmung in einem der Ansprüche 1 bis 5 codiert und ferner einen Terminator umfasst, der die Expression der mindestens einen Nukleinsäuresequenz steuert, und wobei die Wirtszelle ein Mikroorganismus ist, der aus Hefen ausgewählt ist.

9. Wirtszelle nach Anspruch 6 oder 7, wobei das isolierte Nukleinsäuremolekül ferner einen Terminator der Transkription für die Nukleinsäuresequenz umfasst.

10. Wirtszelle nach Anspruch 9, wobei der Transkriptionsterminator ein Hefeterminator ist.

11. Wirtszelle nach einem der Ansprüche 6 bis 10, wobei die Hefe insbesondere aus den Gattungen Saccharomyces, Candida, Ashbya, Dekkera, Pichia (Hansenula), Debaryomyces, Clavispora, Lodderomyces, Yarrowia, Zigosaccharomyces, Schizosaccharomyces, Torulaspora, Kluyveromyces, Brettanomycces, Cryptococcus und Malassezia; insbesondere aus den Arten Saccharomyces cerevisiae, Saccharomyces boulardii, Saccharomyces douglasii, Saccharomyces bayanus, Zigosaccharomyces bailii, Schizosaccharomyces pombe, Dekkera brucelensis, Dekkera intermedia, Brettanomycces custersii, Brettanomycces intermedius, Kluyveromyces themotolerens, Torulaspora globosa und Torulaspora glabrata ausgewählt ist.

12. Wirtszelle nach Anspruch 11, wobei die Gattung der Hefe Saccharomyces ist.

13. Wirtszelle nach Anspruch 12, wobei die Art der Hefe Saccharomyces cerevisiae ist.

14. Verfahren zur Herstellung von Phloroglucinol, das die folgenden Schritte umfasst:
(i) Inkontaktbringen einer Wirtszelle, die das Polypeptid mit Phloroglucinolsynthase-Aktivität gemäß der Begriffsbestimmung in einem der Ansprüche 1 bis 5 exprimiert, beispielsweise einer Wirtszelle nach einem der Ansprüche 6 bis 13, mit einem geeigneten Substrat;
(ii) Anzüchten der Wirtszelle von Schritt (i) *in vitro* unter Bedingungen, die das Wachstum der Wirtszelle und/oder die Expression des Nukleinsäuremoleküls ermöglichen, welches in der Wirtszelle enthalten ist, um auf diese Weise Phloroglucinol herzustellen;
(iii)möglicherweise Gewinnen des Kulturmediums, welches das Phloroglucinol umfasst, wie es nach Schritt (ii) erhalten wurde; und
(iv) möglicherweise Aufreinigen des Phloroglucinols ausgehend von dem Kulturmedium von Schritt (iii).

15. Verfahren zur Herstellung von Phloroglucinol, das zumindest die folgenden Schritte umfasst:
(i) Inkontaktbringen mindestens eines Polypeptids gemäß der Begriffsbestimmung in einem der Ansprüche 1 bis 5 mit einem geeigneten Substrat;
(ii) Inkubieren der Mischung aus Schritt (i) unter Bedingungen, die zur Herstellung von Phloroglucinol geeignet sind;
(iii) möglicherweise Gewinnen des Reaktionsmediums, welches das Phloroglucinol umfasst, wie es nach Schritt (ii) erhalten wurde; und
(iv) möglicherweise Aufreinigen des Phloroglucinols ausgehend von dem Reaktionsmedium von Schritt (iii).

## Claims

1. Use of at least one polypeptide selected from type III polyketide synthases of actinomycete bacteria, as phloroglucinol synthase, said polypeptide comprising at least one amino acid sequence having at least 60% identity with a sequence selected from SEQ ID No.: 4, SEQ ID No.: 1, SEQ NO: 2, SEQ ID No.: 3, SEQ ID No.: 5, SEQ ID.NO: 6, SEQ ID No.: 7, SEQ ID No.: 8, SEQ ID No.: 9 and SEQ ID No.: 10.

2. Use according to Claim 1, said polypeptide comprising at least one amino acid sequence having at least 65% identity with a sequence selected from SEQ ID No.: 4, SEQ ID No.: 1, SEQ NO: 2, SEQ ID No.: 3, SEQ ID No.: 5, SEQ ID.NO: 6, SEQ ID No.: 7, SEQ ID No.: 8, SEQ ID No.: 9 and SEQ ID No.: 10.

3. Use according to Claim 1 or 2, said actinomycete bacteria being selected from the group consisting of *Tsukamurella sp., Nocardia sp., Mycobacterium sp* and *Gordonia sp.,* in particular *Tsukamurella pulmonis, Tsukamurella paurometabola Tsukamurella tyrosinosolvens, Tsukamurella pseudospumae, Tsukamurella sp. 1534, Nocardia farcinica, Mycobacterium marinum, Mycobacterium kansasii, Mycobacterium tuberculosis* and *Gordonia hydrophobica*.

4. Use according to any one of Claims 1 to 3, said polypeptide comprising at least one amino acid sequence having at least 70% identity, more preferably at least 75% identity, more preferably at least 80% identity, even more preferably at least 85% identity, still more preferably at least 90% identity, even more preferentially at least 95% identity, even more preferably at least 96% identity, even more preferably at least 97% identity, still more preferably at least 98% identity, and even more preferentially at least 99% identity, with a sequence selected from SEQ ID No.: 4, SEQ ID No.: 1, SEQ NO: 2, SEQ ID No.: 3, SEQ ID No.: 5, SEQ ID.NO: 6, SEQ ID No.: 7, SEQ ID No.: 8, SEQ ID No.: 9 and SEQ ID No.: 10.

5. Use according to any one of Claims 1 to 4, said polypeptide comprising at least one amino acid sequence selected from SEQ ID No.: 4, SEQ ID No.: 1, SEQ NO: 2, SEQ ID No.: 3, SEQ ID No.: 5, SEQ ID.NO: 6, SEQ ID No.: 7, SEQ ID No.: 8, SEQ ID No.: 9 and SEQ ID No.: 10.

6. Host cell comprising at least one isolated nucleic acid molecule or at least one vector comprising at least one isolated nucleic acid molecule, the isolated nucleic acid molecule comprising at least one nucleic acid sequence encoding a polypeptide as defined in any one of Claims 1 to 5 and further comprising a promoter controlling the expression of said at least one nucleic acid sequence, and the host cell being a microorganism selected from yeasts.

7. Host cell according to Claim 6, the promoter being a yeast promoter.

8. Host cell comprising at least one isolated nucleic acid molecule or at least one vector comprising at least one isolated nucleic acid molecule, the isolated nucleic acid molecule comprising at least one nucleic acid sequence encoding a polypeptide as defined in any one of Claims 1 to 5 and further comprising a terminator controlling the expression of said at least one nucleic acid sequence, and the host cell being a microorganism selected from yeasts.

9. Host cell according to Claim 6 or Claim 7, the isolated nucleic acid molecule further comprising a transcription terminator for said nucleic acid sequence.

10. Host cell according to the claim 9, the transcription terminator being a yeast terminator.

11. Host cell according to any one of the claim 6 to 10, said yeast being in particular selected from the *Saccharomyces*, *Candida, Ashbya*, *Dekkera*, *Pichia* (*Hansenula*), *Debaryomyces*, *Clavispora*, *Lodderomyces*, *Yarrowia*, *Zigosaccharomyces, Schizosaccharomyces*, *Torulaspora*, *Kluyveromyces*, *Brettanomycces*, *Cryptococcus* and *Malassezia* genera; more particularly from the species *Saccharomyces cerevisiae*, *Saccharomyces boulardii, Saccharomyces douglasii, Saccharomyces bayanus*, *Zigosaccharomyces bailii, Schizosaccharomyces pombe, Dekkera brucelensis, Dekkera intermedia, Brettanomycces custersii, Brettanomycces intermedius, Kluyveromyces themotolerens, Torulaspora globosa* and *Torulaspora glabrata*.

12. Host cell according to claim 11, said yeast being of the genus *Saccharomyces.*

13. Host cell according to claim 12, said yeast being of the species *Saccharomyces cerevisiae*

14. Method for producing phloroglucinol, comprising the steps consisting in:
(i) bringing a host cell expressing the polypeptide with phloroglucinol synthase activity as defined in any one of Claims 1 to 5, for example a host cell according to any one of Claims 6 to 13, into contact with a suitable substrate;
(ii) culturing, *in vitro*, the host cell of step (i) under conditions which allow the growth of said host cell and/or the expression of the nucleic acid molecule contained in said host cell, so as to produce phloroglucinol;
(iii) optionally, recovering the culture medium comprising the phloroglucinol, obtained after step (ii); and
(iv) optionally, purifying the phloroglucinol from the culture medium of step (iii).

15. Method for producing phloroglucinol, comprising at least the steps consisting in:
(i) bringing at least one polypeptide as defined in any one of Claims 1 to 5 into contact with a suitable substrate;
(ii) incubating the mixture resulting from step (i) under conditions suitable for producing phloroglucinol;
(iii) optionally, recovering the reaction medium comprising the phloroglucinol, obtained after step (ii); and
(iv) optionally, purifying the phloroglucinol from the reaction medium of step (iii).
